# EUROPEAN PATENT APPLICATION

(11) **EP 4 001 413 A1**
(43) Date of publication of application: **25.05.2022**
(21) Application number: 20843810.1
(22) Date of filing: 16.07.2020
(51) Int. Cl.: C12N 15/12, C12Q 1/6837, C12Q 1/686, C12Q 1/6869, C12Q 1/6886

(54) **INTERNAL STANDARD GENE**

(30) Priority: 19.07.2019 JP 2019134182
(71) Applicant: Public University Corporation Fukushima Medical University, Fukushima City Fukushima, 960-1295 (JP)
(72) Inventor: WATANABE Shinya, Fukushima City Fukushima 9601295 (JP); IMAI Junichi, Fukushima City Fukushima 9601295 (JP); ITO Emi, Fukushima City Fukushima 9601295 (JP); OTAKE Toru, Fukushima City Fukushima 9601295 (JP); ABE Noriko, Fukushima City Fukushima 9601295 (JP); TACHIBANA Kazunoshin, Fukushima City Fukushima 9601295 (JP)
(74) Representative: Betten & Resch
(86) International application number: PCT/JP2020/027650
(87) International publication number: WO 2021/015085

(57) **Abstract**

An object of the present invention is to provide a novel internal standard gene for gene expression analysis and to provide a gene expression analysis method using the internal standard gene. The present invention provides a gene expression analysis method for a test sample, comprising the steps of: (a) measuring an expression level of a desired gene; (b) measuring at least one internal standard gene selected from the group consisting of ABCF3, FBXW5, MLLT1, FAM234A, PITPNM1, WDR1, NDUFS7, and AP2A1; and (c) comparing the expression level of the desired gene with the expression level of the internal standard gene.

## Description

### [Technical Field]

The present invention relates to an internal standard gene. Particularly, the present invention relates to an internal standard gene that is used in measuring the expression quantity or expression level of a gene of interest present in a biological sample (including a breast cancer tissue and a normal mammary gland tissue) derived from a breast cancer patient.

### [Background Art]

Gene expression analysis is one of the approaches of comparing two or more different biological samples and detecting their difference. The gene expression analysis involves extracting RNA contained in each biological sample and measuring the expression quantity or expression level of a particular gene therefrom for comparison. In the case of performing gene expression analysis by Northern blot, RT-PCR, real-time PCR, or the like, the comparison of the expression quantity or expression level of a particular gene is not sufficient and it is necessary to compare this expression quantity or expression level as a relative value to the expression quantity or expression level of a gene called internal standard gene. This is because, for example, (1) the amounts of biological samples to be compared are difficult to accurately adjust to the same amounts, and RNA extraction efficiency differs among biological samples even if the biological samples are used in the same amounts; and (2) the ratio of mRNA contained in RNA differs among biological samples even if RNA levels are the same. Thus, relative comparison with the internal standard gene has heretofore been required. For example, glyceraldehyde-3-phosphate dehydrogenase (GAPDH) gene and β actin (ACTB) gene known as so-called housekeeping genes have been traditionally used as the internal standard gene. These housekeeping genes are constitutively expressed in order to maintain the vital activities of cells themselves, and expressed at a constant level, irrespective of the type of cells or tissues. Therefore, the housekeeping genes are used on the assumption that they are not changed by experimental treatment.

However, the fact has become known that the expression of the internal standard gene differs depending on the type of cells or tissues and is adjusted by experimental conditions, the stages of development, etc. In actuality, there is a risk of misinterpretation ascribable to a selected internal standard gene whose expression quantity may vary depending on experimental conditions, etc., for gene expression analysis. Many researchers have also pointed out concerns about this. An internal standard gene that can be used universally, irrespective of particular tissues or experimental conditions, and usefully in normalization for gene expression analysis has already been searched for (Patent Literature 1). In such studies, however, search has often been made using gene expression data registered in an existing public database. Furthermore, such a public database includes the admixture of data measured under different platforms in a plurality of different facilities. Therefore, simple parallel comparison is actually difficult. On the other hand, there is also a study to search for a proper internal standard gene in a closed system dedicated to a particular experiment (Patent Literature 2). The internal standard gene thus searched for is selected in many cases as a result of verification based on data measured under the same platform in the same facility, and is therefore highly reliable as long as the internal standard gene is used in the system.

Breast cancer is variable and is classified into a plurality of subtypes having various features. This subtype classification was originally made by exhaustive gene expression analysis (Non Patent Literature 1). Since prognosis or drug sensitivity differs depending on this classification, this classification serves as an index for selecting medication. In actual clinical practice, diagnosis is generally performed using a convenient immunohistochemical approach, though not a few cases have breast cancer different from classification by gene expression analysis. Thus, there is a demand for highly accurate examination techniques by gene expression analysis. Research on breast cancer has a long history, and a large number of studies have been conducted by gene expression analysis using cell lines derived from breast cancer. Internal standard genes support the reliability of such examination techniques or studies.

### [Citation List]

### [Patent Literature]

[Patent Literature 1] Japanese Patent No. 5934036
[Patent Literature 2] Japanese Patent Laid-Open No. 2012-105614

### [Non Patent Literature]

[Non Patent Literature 3] Perou CM, Sorlie T, Eisen MB, et al., Molecular portraits of human breast tumours. Nature 406: 747-752, 2000

### [Summary of Invention]

### [Technical Problem]

An object of the present invention is to provide a novel internal standard gene for gene expression analysis and to provide a gene expression analysis method using the internal standard gene. Particularly, an object of the present invention is to provide a gene capable of serving as an internal standard gene in the comparative analysis of samples derived from breast cancer, and a gene expression analysis method for a breast cancer-derived sample using the gene.

### [Solution to Problem]

In order to attain the object, the present inventors have obtained gene expression profiles of 14,400 genes from each of specimens of 470 cases in total involving breast cancer tissues (453 cases) and normal mammary gland tissues (17 cases), and successfully selected internal standard genes for the gene expression analysis of living tissues (including normal mammary gland tissues) derived from breast cancer, leading to the present invention.

Specifically, the present invention includes the following aspects.

In one aspect, the present invention relates to
[1] a gene expression analysis method for a test sample, comprising the steps of:
   (a) measuring an expression level of a desired gene;
   (b) measuring at least one internal standard gene selected from the group consisting of ABCF3, FBXW5, MLLT1, FAM234A, PITPNM1, WDR1, NDUFS7, and AP2A1; and
   (c) normalizing the expression level of the desired gene using the expression level of the internal standard gene.

In this context, in one embodiment, the gene expression analysis method of the present invention is [2] the gene expression analysis method according to [1], wherein
the test sample is a sample derived from a breast cancer patient.

In one embodiment, the gene expression analysis method of the present invention is
the gene expression analysis method according to [2], wherein
the desired gene is a gene for identifying or classifying a subtype of breast cancer.

In another aspect, the present invention relates to [4] an internal standard gene for gene expression analysis consisting of at least one gene selected from the group consisting of FBXW5, PITPNM1, MLLT1, WDR1, ABCF3, NDUFS7, FAM234A, and AP2A1.

In this context, in one embodiment, the internal standard gene of the present invention is
the internal standard gene according to [4], wherein
the internal standard gene is used in gene expression analysis for a test sample derived from a breast cancer patient.

In one embodiment, the internal standard gene of the present invention is
the internal standard gene according to [5], wherein
the gene expression analysis for a test sample derived from a breast cancer patient is gene expression analysis for identifying a subtype of breast cancer.

In an alternative aspect, the present invention relates to
a composition for expression analysis of an internal standard gene, comprising a unit for measuring an expression level of an internal standard gene according to [4] .

In this context, in one embodiment, the composition for expression analysis of an internal standard gene of the present invention relates to
a composition for expression analysis of an internal standard gene for identifying or classifying breast cancer, comprising a unit for measuring an expression level of an internal standard gene according to [5] or [6] .

In this context, in one embodiment, the composition for expression analysis of an internal standard gene for identifying or classifying breast cancer according to the present invention is [9] the composition for expression analysis of an internal standard gene for identifying or classifying breast cancer according to [8], wherein
the unit for measuring an expression level of the gene is at least one unit selected from the group consisting of a primer, a probe, and an antibody against the gene, and labeled forms thereof.

In one embodiment, the composition for expression analysis of an internal standard gene for identifying or classifying breast cancer according to the present invention is [10] the composition for expression analysis of an internal standard gene for identifying or classifying breast cancer according to [8] or [9], wherein
the composition is intended for PCR, a microarray, or RNA sequencing.

### [Advantageous Effects of Invention]

The internal standard gene of the present invention provides a novel internal standard gene that can be used in gene expression analysis. A gene expression analysis method using the internal standard gene can also be provided. The internal standard gene of the present invention substantially rarely varies in expression level, without being influenced by the subtype of breast cancer in a sample derived from a breast cancer patient, and differs only slightly in expression level relative to the expression level of a human common reference. Accordingly, the internal standard gene of the present invention is useful, particularly, in the gene expression analysis of a sample derived from breast cancer.

### [Brief Description of Drawings]

[Figure 1] Figure 1 is a graph showing the distributions of expression levels of four genes (ABCF3, MLLT1, FBXW5, and FAM234A) in the gene expression profiles of 470 samples measured in Example 3 described below.
[Figure 2] Figure 2 is a graph showing the distributions of expression levels of four genes (PITPNM1, NDUFS7, WDR1, and AP2A1) in the gene expression profiles of 470 samples measured in Example 3 described below.
[Figure 3] Figure 3 is a graph showing the distribution of expression levels of GAPDH gene in the gene expression profiles of 470 samples measured in Example 3 described below.
[Figure 4] Figure 4 shows a heatmap of results of conducting the cluster analysis of a gene averaging technique based on a Euclidean distance as to 470 cases using a set of 207 identification marker genes including internal standard genes shown in Example 4 described below.

### [Description of Embodiments]

### 1. Internal standard gene

### 1-1. Summary

The first aspect of the present invention is an internal standard gene that can be used in gene expression analysis.

The internal standard gene according to the present invention consists of at least one gene selected from the group consisting of FBXW5, PITPNM1, MLLT1, WDR1, ABCF3, NDUFS7, FAM234A, and AP2A1.

### 1-2. Definition

The "internal standard gene" is a gene that is used in normalization for relatively indicating the amount of a particular gene in gene expression analysis. The internal standard gene according to the present invention is eight genes, FBXW5, PITPNM1, MLLT1, WDR1, ABCF3, NDUFS7, FAM234A, and AP2A1, as described above. The following table shows the symbols, gene names, and reference sequence IDs (RefSeq IDs) registered in the NCBI database, of these internal standard genes.

**[Table 1]**

| Symbol | Name | ID | SEQ ID NO |
|---|---|---|---|
| FBWX5 | F-box and WD-40 domain protein 5 (FBXW5), transcript variant 2, mRNA | NM_018998 | SEQ ID NO: 1 |
| PITPNM1 | phosphatidylinositol transfer protein, membrane-associated 1 (PITPNM1), mRNA | NM_004910 | SEQ ID NO: 2 |
| MLLT1 | myeloid/lymphoid or mixed lineage leukemia (trithorax homolog, Drosophila): translocated to, 1 (MLLT1), mRNA | NM_005934 | SEQ 1D NO: 3 |
| WDR1 | WD repeat domain 1 (NNDR1), transcript variant 1, mRNA | NM_017491 | SEQ ID NO: 4 |
| ABCF3 | ATP-binding cassette, sub-family F (GCN20). member 3 (ABCF3), mRNA. | NM_018358 | SEQ ID NO: 5 |
| NDUFS7 | NADH dehydrogenase (ubiquinone) Fe·S protein 7, 20kDa (NADH-coenzyme Q reductase) (NDUFS7), mRNA. | NM_024407 | SEQ ID NO: 6 |
| FAM234A | hypothetical protein DKFZp761D0211 (DKFZP761D0211), mRNA | NM_032039 | SEQ ID NO: 7 |
| AP2A1 | adaptor-related protein complex 2, alpha 1 subunit (AP2A1), transcript variant 2, mRNA | NM_130787 | SEQ I D NO: 8 |

The internal standard gene according to the present invention (FBXW5, PITPNM1, MLLT1, WDR1, ABCF3, NDUFS7, FAM234A, and AP2A1) can be defined as a gene (or a polynucleotide) having the nucleotide sequence represented by each of SEQ ID NOs: 1 to 8.

In one embodiment, the internal standard gene according to the present invention is at least one gene selected from the group consisting of a gene consisting of the nucleotide sequence represented by SEQ ID NO: 1, a gene consisting of the nucleotide sequence represented by SEQ ID NO: 2, a gene consisting of the nucleotide sequence represented by SEQ ID NO: 3, a gene consisting of the nucleotide sequence represented by SEQ ID NO: 4, a gene consisting of the nucleotide sequence represented by SEQ ID NO: 5, a gene consisting of the nucleotide sequence represented by SEQ ID NO: 6, a gene consisting of the nucleotide sequence represented by SEQ ID NO: 7, and a gene consisting of the nucleotide sequence represented by SEQ ID NO: 8.

In the present specification, the FBXW5, PITPNM1, MLLT1, WDR1, ABCF3, NDUFS7, FAM234A, and AP2A1 genes also include, for example, genes consisting of a nucleotide sequence containing a degenerate codon and encoding the same amino acid sequence, mutated genes, such as various variants and point mutant genes, of each gene, and ortholog genes of organisms of other species such as chimpanzees. Such genes include genes consisting of a polynucleotide consisting of a nucleotide sequence having 70% or higher (preferably 75% or higher, 80% or higher, or 85% or higher, more preferably 90% or higher, 95% or higher, 96% or higher, 97% or higher, 98% or higher, or 99% or higher) base identity to the nucleotide sequence defined in any of SEQ ID NOs: 1 to 8, the polynucleotide maintaining the functions of the intended gene.

In one embodiment, for example, the ABCF3 gene used in the present invention can be defined as a gene (or a polynucleotide) consisting of the nucleotide sequence represented by SEQ ID NO: 1. In this respect, the ABCF3 gene includes a gene consisting of a polynucleotide consisting of a nucleotide sequence having 70% or higher (preferably 75% or higher, 80% or higher, or 85% or higher, more preferably 90% or higher, 95% or higher, 96% or higher, 97% or higher, 98% or higher, or 99% or higher) base identity to the nucleotide sequence represented by SEQ ID NO: 1, the polynucleotide maintaining the functions of the ABCF3 gene. In the present specification, the "base identity" refers to the ratio (%) of the number of matched bases in the nucleotide sequences of nucleotides to be compared to the total number of bases in the genes when the two nucleotide sequences are aligned and a gap is introduced thereto, if necessary, so as to attain the highest degree of base matching between the nucleotide sequences.

In the present specification, the phrase "gene consisting of the nucleotide sequence represented by particular SEQ ID NO" also includes a polynucleotide hybridizing under stringent conditions to a nucleotide fragment consisting of a nucleotide sequence complementary to a partial nucleotide sequence of the gene, the polynucleotide maintaining the functions of the intended gene. The "stringent conditions" mean conditions under which any nonspecific hybrid is not formed. In general, more highly stringent conditions involve a lower salt concentration and a higher temperature. Low stringent conditions are, for example, conditions under which washing is performed with 1 × SSC and 0.1% SDS at approximately 37°C in washing after hybridization, and more stringent conditions are conditions under which washing is performed with 0.5 x SSC and 0.1% SDS at approximately 42°C to 50°C. Highly stringent conditions, which are much stricter, are, for example, conditions under which washing is performed with 0.1 x SSC and 0.1% SDS at 50°C to 70°C, 55°C to 68°C, or 65°C to 68°C in washing after hybridization. In general, highly stringent conditions are preferred. The combinations of the SSC, the SDS and the temperature described above are mere illustrations. Those skilled in the art may determine the stringency of hybridization by appropriately combining the SSC, the SDS and the temperature as well as other conditions such as a probe concentration, a probe base length, and a hybridization time. In a preferred embodiment, the polynucleotide hybridizing under stringent conditions to a nucleotide fragment consisting of a nucleotide sequence complementary to a partial nucleotide sequence of a gene consisting of the nucleotide sequence represented by particular SEQ ID NO is a polynucleotide consisting of a nucleotide sequence having 70% or higher (preferably 75% or higher, 80% or higher, or 85% or higher, more preferably 90% or higher, 95% or higher, 96% or higher, 97% or higher, 98% or higher, or 99% or higher) base identity to the nucleotide sequence represented by the particular SEQ ID NO.

In the present specification, the term "internal standard gene" includes a gene defined by a DNA sequence as well as a gene product such as a transcript (mRNA and cDNA) and a translation product (protein) based on the gene.

At least one internal standard gene may be selected and used as the internal standard gene according to the present invention, or two or more internal standard genes may be used in combination. In the case of combining two or more internal standard genes, the internal standard genes may be selected from the group consisting of FBXW5, PITPNM1, MLLT1, WDR1, ABCF3, NDUFS7, FAM234A, and AP2A1, or any of these genes may be combined with an internal standard gene other than the group.

Examples of the internal standard gene other than the group consisting of FBXW5, PITPNM1, MLLT1, WDR1, ABCF3, NDUFS7, FAM234A, and AP2A1 can include commercially available universal references, housekeeping genes known in the art (e.g., glyceraldehyde-3-phosphate dehydrogenase (GAPDH) gene and β actin (ACTB) gene) and combinations thereof. Those skilled in the art can select an internal standard gene suitable for conditions of each gene expression analysis through a test and can also use the selected internal standard gene together with the internal standard gene according to the present invention.

In one embodiment, the internal standard gene according to the present invention is an isolated gene (or gene product). In the present specification, the term "isolated" refers to a gene (or a gene product) isolated from a living body in the broad sense and includes a product obtained by substantially removing a factor naturally accompanying a gene (or a gene product) in the narrow sense.

The internal standard gene according to the present invention varies only slightly in the expression level of the gene among test samples derived from healthy individuals and patients having any subtype of breast cancer.

Accordingly, in one embodiment, the internal standard gene of the present invention can be used in gene expression analysis for a test sample derived from a breast cancer patient. In this context, the gene expression analysis for a test sample derived from a breast cancer patient includes, for example, gene expression analysis for identifying the presence or absence of breast cancer, and gene expression analysis for identifying or classifying a subtype of breast cancer. In this context, the term "identifying or classifying" refers to identifying the presence of breast cancer, identifying the high or low possibility that breast cancer is present, identifying or classifying any subtype to which breast cancer belongs, or identifying or classifying the high or low possibility that breast cancer belongs to any tissue type, as to a sample derived from a test subject having a history of breast cancer.

Use of the internal standard gene according to the present invention in the gene expression analysis for a test sample derived from a breast cancer patient enables a more accurate relative value of gene expression to be provided by comparison with an internal standard gene known in the art (e.g., GAPDH) or the like.

In the present specification, the "test sample" refers to a sample that is used in gene expression analysis. The test sample that can be used in the present invention is not particularly limited as long as the sample expresses the internal standard gene according to the present invention. Examples of the animal from which the test sample is derived can include humans, monkeys, and chimpanzees. A human is preferred. Examples of the "sample" can include tissue, cells, body fluids (blood (including serum, plasma and interstitial fluid), spinal fluid (cerebrospinal fluid), urine, lymph, digestive fluid, ascetic fluid, pleural effusion, fluid around the nerve root, extracts from each tissue or cell, etc.) and samples collected from living bodies, such as peritoneal lavages, cultured cells, and purified or prepared products thereof.

In the case of using the internal standard gene according to the present invention in the identification of breast cancer by gene expression analysis, the "test sample" is a sample collected from a human test subject and preferably contains a breast cancer tissue or a tissue suspected of being a breast cancer tissue, or a portion thereof. In this context, the "tissue" and the "cell" may be derived from any site of a test subject and is preferably a specimen collected by biopsy or surgically excised, more specifically, a breast tissue or a breast cell. A breast cancer cell collected by biopsy or a breast cancer tissue or a breast cancer cell suspected of having breast cancer is particularly preferred. Such a tissue or a cell may be formalin-fixed paraffin embedded (FFPE).

The "breast cancer" refers to a cancer that usually develops from the breast duct or a tissue within the breast duct, such as the lobule. The breast cancer includes carcinoma and sarcoma and refers to every malignant tumor of a breast tissue. The breast cancer is a heterogeneous disease and is classified into a plurality of subtypes having various features.

Subtypes of breast cancer are mainly classified into 11 types, luminal A, luminal B (HER2-positive), luminal B (HER2-negative), HER2-positive, HER2-positive-like, triple negative, phyllodes tumor, squamous cell cancer, indeterminable, normal-like, and normal.

In this context, the term "luminal A" clinicopathologically refers to a case that satisfies all of 1) ER positivity and PgR negativity, 2) HER2 negativity, 3) a low level of Ki67, and 4) a low risk of recurrence in MEGA. In the present specification, the term also includes cases similar in gene expression profiles to most of cases clinicopathologically diagnosed with luminal A.

The clinicopathological diagnosis of subtypes mainly involves, but is not limited to, confirming the expression of ER, PgR, HER2, and Ki67 by immunohistological staining, and also includes the confirmation thereof by gene expression analysis.

The term "luminal B (HER2-positive)" clinicopathologically refers to an ER-positive and HER2-positive case. In the present specification, the term also includes cases similar in gene expression profiles to most of cases clinicopathologically diagnosed with luminal B (HER2-positive).

The term "luminal B (HER2-negative)" clinicopathologically refers to a case that falls into any of 1) ER positivity and HER2 negativity, 2) a high level of Ki67, and 3) PgR negativity or a low level of PgR, and 4) a high risk of recurrence in MEGA. In the present specification, the term also includes cases more highly expressing a cell cycle-related gene group than other cases among luminal A cases.

The term "HER2-positive" clinicopathologically refers to a HER2-positive, ER-negative and PgR-negative case. In the present specification, the term also includes cases similar in gene expression profiles to most of cases clinicopathologically diagnosed with HER2-positive.

The term "HER2-positive-like" refers to a case that is HER2-negative but is similar in the other gene expression profiles to most of cases clinicopathologically diagnosed with HER2-positive.

The term "triple negative" clinicopathologically refers to an ER-negative, PgR-negative and HER2-negative case. In the present specification, the term also includes cases similar in gene expression profiles to most of cases clinicopathologically diagnosed with triple negative.

The term "squamous cell cancer" is a cancer caused by the malignant proliferation of cells called epidermal keratinocytes present in the epidermis. In the present specification, the term refers to a cancer that originates in the mammary gland.

The term "phyllodes tumor" is clinicopathologically similar to mammary fibroadenoma and refers to a tumor having the rapid proliferation of fibrous stroma and breast duct epithelium with respect to fibrous tumor in which intralobular connective tissues of the mammary gland proliferate.

The term "indeterminable" refers to a case that is not similar in gene expression profiles to any of luminal A, luminal B (HER2-positive), luminal B (HER2-negative), HER2-positive, HER2-positive-like, triple negative, normal-like, normal, squamous cell cancer and phyllodes tumor.

The term "normal-like" refers to a case that is clinicopathologically diagnosed with "cancer" but is similar in gene expression profiles to normal mammary gland tissues.

The term "normal" refers to a normal tissue.

The internal standard gene according to the present invention can be suitably used in gene expression analysis for identifying the subtypes of breast cancer listed above.

### 2. Composition for gene expression analysis

### 2-1. Summary

In another aspect, the present invention relates to a composition for expression analysis of an internal standard gene, comprising a unit for measuring an expression level of the internal standard gene.

### 2-2. Definition

In the present specification, the "expression level of a gene" refers to the amount of a transcript, expression intensity or expression frequency of the gene. In this context, the expression level of a gene may include not only the expression level of the wild-type gene of the gene but the expression level of a mutated gene such as a point mutant gene. The transcript that indicates the expression of the gene may also include variant transcripts such as splicing variants and fragments thereof. This is because even information based on such a mutated gene, a transcript, or a fragment thereof permits use as the internal standard gene according to the present invention. The expression level of a gene can be obtained as a measurement value by the measurement of the amount of a transcript, i.e., a mRNA level, a cDNA level, etc., of the gene. In a preferred embodiment, the measurement of the expression level of a gene is the measurement of mRNA.

In the present specification, the "unit for measuring an expression level" is a compound that can bind to a gene transcript, and is a compound that can indicate the presence or absence of the transcript or the amount of the transcript. In one embodiment, the unit for measuring an expression level is at least one compound selected from the group consisting of a primer and a probe against the gene to be measured, and labeled forms thereof.

The primer or the probe that can be used in the present invention is usually constituted by a natural nucleic acid such as DNA or RNA. Highly safe, easy-to-synthesize, and inexpensive DNA is particularly preferred. If necessary, the natural nucleic acid may be combined with a chemically modified nucleic acid or a pseudo nucleic acid. Examples of the chemically modified nucleic acid or the pseudo nucleic acid include PNA (peptide nucleic acid), LNA (Locked Nucleic Acid(R)), methyl phosphonate-type DNA, phosphorothioate-type DNA, and 2'-O-methyl-type RNA. The primer or the probe can be labeled or modified with a labeling material such as a fluorescent material and/or a quencher material, or a radioisotope (e.g., 32P, 33P, and 35S), or a modifying material such as biotin or (strept)avidin, or magnetic beads, and used as a labeled form. The labeling material is not limited, and a commercially available product can be used. For example, a fluorescent material such as FITC, Texas, Cy3, Cy5, Cy7, cyanine 3, cyanine 5, cyanine 7, FAM, HEX, VIC, fluorescamine or a derivative thereof, or rhodamine or a derivative thereof can be used. A quencher material such as AMRA, DABCYL, BHQ-1, BHQ-2, or BHQ-3 can be used. The labeling position of the labeling material in the primer or the probe can be appropriately determined according to the characteristics of the modifying material or intended use. In general, a 5'- or 3'-terminal portion is often modified. One primer or probe molecule may be labeled with one or more labeling materials. The labeling of a nucleotide with such a material can be performed by a method known in the art.

A nucleotide for use as the primer or the probe may be any nucleotide composed of the sense strand or antisense strand of the gene to be measured. In a preferred embodiment, the nucleotide for use as the primer or the probe is a primer or a probe for PCR, for a microarray, or for RNA sequencing.

The base length of the primer or the probe is not particularly limited as long as the expression level of the gene of interest can be measured. The probe has at least a 10-base length or more to the full length of the gene, preferably a 15-base length or more to the full length of the gene, more preferably a 30-base length or more to the full length of the gene, further preferably a 50-base length or more to the full length of the gene, for use in a hybridization technique mentioned later, and has a 10- to 200-base length, preferably a 20- to 150-base length, more preferably a 30- to 100-base length, for use in a microarray. In general, a longer probe elevates hybridization efficiency and enhances sensitivity. On the other hand, a shorter probe reduces sensitivity but rather elevates specificity. The primer may be 10 to 50 bp, preferably 15 to 30 bp each of a forward primer and a reverse primer.

In the case of measuring the expression level of the internal standard gene according to the present invention by a nucleic acid amplification technique or the like, for example, probes shown in SEQ ID NOs: 9 to 16 can be used for FBXW5, PITPNM1, MLLT1, WDR1, ABCF3, NDUFS7, FAM234A, and AP2A1, respectively (Table 2), though the probe is not limited thereto. The preparation of the primer or the probe for each approach of gene expression analysis is known to those skilled in the art, and the primer or the probe can be prepared in accordance with, for example, a method described in Greene & Sambrook, Molecular Cloning (2012) mentioned above. It is also possible to provide sequence information to a nucleic acid synthesis commissioned manufacturer, which in turn produces the primer or the probe on consignment.

**[Table 2]**

| **Symbol** | Sequence ID | Sequence of probe | SEQ ID NO |
|---|---|---|---|
| FBXW5 | NM_018998 | ACCACTGGCTGCCTCACCTACTCCCCACACCAGATCGGCATCA AGCAGATCCTGCCACACCAGATGACCACGGCAGGGCC | SEQ ID NO: 9 |
| PITPNM1 | NM_004910 | CACTCCAGCCTCTTTCTGGAGGAGCTGGAGATGCTGGTGCCCT CAACACCCACCTCTACTAGCGGTGCCTTCTGGAAGGG | SEQ ID NO: 10 |
| MLLT1 | NM_005934 | ATCTGATCGAGGAGACTGGCCACTTCAATGTCACCAACACCAC CTTCGACTTCGACCTCTTCTCCCTGGACGAGACCACC | SEQ ID NO: 11 |
| WDR1 | NM_017491 | AGCCTGGCCTGGCTGGACGAGCACACGCTGGTCACGACCTCC CATGATGCCTCTGTCAAGGAGTGGACAATCACCTACTG | SEQ ID NO: 12 |
| ABCF3 | NM_018358 | TGACTATGCCCTGCCCCAACTTCTACATTCTGGATGAACCCAC AAACCACCTGGACATGGAGACCATTGAGGCTCTGGGC | SEQ ID NO: 13 |
| NDUFS7 | NM_024407 | ACTATTCCTACTCGGTGGTGAGGGGCTGCGACCGCATCGTGCC CGTGGACATCTACATCCCAGGCTGCCCACCTACGGCC | SEQ ID NO: 14 |
| FAM234A | NM_032039 | TGGCACCGACAGACAGATCCTGTTTCTGGACCTTGGCACTGGA GCCGTCCTGTGTAGCCTAGCCCTCCCGAGCCTCCCTG | SEQ ID NO: 15 |
| AP2A1 | NM_130787 | AGCATTCCAACGCCAAGAACGCCATCCTCTTCGAGACCATCAG CCTCATCATCCACTATGACAGTGAGCCCAACCTCCTG | SEQ ID NO: 16 |

When the unit for measuring an expression level is any of the probes as described above, each probe may be provided in the form of a DNA microarray or a DNA microchip in which the probe is immobilized on a substrate. The material of the substrate for immobilizing each probe thereon is not limited, and, for example, a glass plate, a quartz plate, or a silicon wafer is usually used. Examples of the size of the substrate include 3.5 mm × 5.5 mm, 18 mm × 18 mm, and 22 mm x 75 mm, which can be variously set according to the number of probe spots, the size of the spots, etc. For the probe, 0.1 µg to 0.5 µg of a nucleotide is usually used per spot. Examples of the nucleotide immobilization method include a method of electrostatically binding the nucleotide through the use of its charge to a solid-phase support surface-treated with a polycation such as polylysine, poly-L-lysine, polyethyleneimine, or polyalkylamine, and a method of covalently binding the nucleotide harboring a functional group such as an amino group, an aldehyde group, a SH group, or biotin to solid-phase surface harboring a functional group such as an amino group, an aldehyde group, or an epoxy group.

The composition for expression analysis of an internal standard gene of the present invention may be provided as a kit comprising other reagents (e.g., probes or primers against other genes, or labeled forms thereof, or a buffer) or an instrument (culture dish, etc.) necessary for the measurement or detection of gene expression, and an instruction for use in the identification of breast cancer.

### 3. Gene expression analysis method using internal standard gene

### 3-1. Summary

In an alternative aspect, the present invention relates to a gene expression analysis method for a test sample using the internal standard gene according to the present invention. The gene expression analysis method comprises the steps of:
(a) measuring an expression level of a desired gene;
(b) measuring at least one internal standard gene selected from the group consisting of ABCF3, FBXW5, MLLT1, FAM234A, PITPNM1, WDR1, NDUFS7, and AP2A1; and
(c) normalizing the expression level of the desired gene using the expression level of the internal standard gene.

### 3-2. Definition

The gene expression analysis method according to the present invention comprises the step of (a) measuring an expression level of the desired gene.

The "step of measuring an expression level of the gene" is the step of measuring an expression level of the gene in a test sample to obtain a measurement value thereof.

The measurement of the expression level of the gene is preferably the measurement of the expression level per unit amount. In the present specification, the "unit amount" refers to an arbitrarily set amount of a sample. For example, a volume (indicated by µL or mL) or a weight (indicated by µg, mg, or g) corresponds thereto. The unit amount is not particularly defined, and the unit amount to be measured by a series of procedures in the gene expression analysis method is preferably constant.

In the present specification, the "desired gene" refers to a gene whose relative value of a gene expression level is to be examined using the internal standard gene according to the present invention. The desired gene is not particularly limited as long as the gene is expressed in the same cells as those expressing the internal standard gene according to the present invention. Those skilled in the art can appropriately select the desired gene according to the purpose of gene expression analysis. In one embodiment, the desired gene is a gene for identifying breast cancer. The gene for identifying breast cancer is a gene whose expression level varies specifically in breast cancer. Accordingly, in the embodiment, the desired gene can be a gene known to specifically exhibit variations in expression level in breast cancer. In a more preferred embodiment, the desired gene is a gene for identifying or classifying a subtype of breast cancer.

Examples of the gene for identifying or classifying a subtype of breast cancer can include, but are not limited to, ABCF3 gene, FBXW5 gene, MLLT1 gene, FAM234A gene, PITPNM1 gene, WDR1 gene, NDUFS7 gene, AP2A1 gene, KRTDAP gene, SERPINB3 gene, SPRR2A gene, SPRR1B gene, KLK13 gene, KRT1 gene, LGALS7 gene, PI3 gene, SERPINH1 gene, SNAI2 gene, GPR173 gene, HAS2 gene, PTH1R gene, PAGE5 gene, ITLN1 gene, SH3PXD2B gene, TAP1 gene, FN1 gene, CTHRC1 gene, MMP9 gene, ADIPOQ gene, CD36 gene, G0S2 gene, GPD1 gene, LEP gene, LIPE gene, PLIN1 gene, SDPR gene, LIFR gene, TGFBR3 gene, CAPN6 gene, PIGR gene, KRT15 gene, KRT5 gene, KRT14 gene, DST gene, WIF1 gene, SYNM gene, KIT gene, GABRP gene, SFRP1 gene, ELF5 gene, MIA gene, MMP7 gene, FDCSP gene, CRABP1 gene, PROM1 gene, KRT23 gene, S100A1 gene, WIPF3 gene, CYYR1 gene, TFCP2L1 gene, DSC2 gene, MFGE8 gene, KLK7 gene, KLK5 gene, DSG3 gene, TTYH1 gene, SCRG1 gene, S100B gene, ETV6 gene, OGFRL1 gene, MELTF gene, HORMAD1 gene, PKP1 gene, FOXC1 gene, ITGB8 gene, VGLL1 gene, ART3 gene, EN1 gene, SPHK1 gene, TRIM47 gene, COL27A1 gene, RFLNA gene, RASD2 gene, A2ML1 gene, MARCO gene, TSPYL5 gene, TM4SF1 gene, FABP5 gene, SPIB gene, BCL2A1 gene, MZB1 gene, KCNK5 gene, LMO4 gene, RNF150 gene, LYZ gene, C21orf58 gene, ATP13A5 gene, NUDT8 gene, HSD17B2 gene, ABCA12 gene, ENPP3 gene, WNT5A gene, MPP3 gene, VPS13D gene, PXMP4 gene, GGT1 gene, TRPV6 gene, C2orf54 gene, CLDN8 gene, LBP gene, SRD5A3 gene, PAPSS2 gene, TMEM45B gene, CLCA2 gene, FASN gene, MPHOSPH6 gene, NXPH4 gene, HPGD gene, KYNU gene, GLYATL2 gene, KMO gene, SRPK3 gene, THRSP gene, PLA2G2A gene, TFAP2B gene, FABP7 gene, SLPI gene, SERHL2 gene, S100A9 gene, KRT7 gene, TMEM86A gene, MBOAT1 gene, PGAP3 gene, STARD3 gene, ERBB2 gene, MIEN1 gene, GRB7 gene, GSDMB gene, ORMDL3 gene, MED24 gene, MSL1 gene, CASC3 gene, WIPF2 gene, THSD4 gene, MAPT gene, LONRF2 gene, TCEAL3 gene, DBNDD2 gene, FGD3 gene, GFRA1 gene, PARD6B gene, STC2 gene, SLC39A6 gene, ENPP5 gene, ZNF703 gene, EVL gene, TBC1D9 gene, CHAD gene, GREB1 gene, HPN gene, IL6ST gene, FAM198B gene, CA12 gene, KCNE4 gene, NAT1 gene, CYP2B6(CYP2B7P) gene, ARMT1 gene, MAGED2 gene, CELSR1 gene, INPP5J gene, PADI2 gene, PPP1R1B gene, ESR1 gene, MLPH gene, FOXA1 gene, XBP1 gene, GATA3 gene, ZG16B gene, KIAA0040 gene, TMC4 gene, AGR2 gene, TFF3 gene, SCGB2A2 gene, MUCL1 gene, DDX11 gene, ATAD2 gene, GGH gene, CDCA3 gene, CCNA2 gene, CCNB2 gene, ANLN gene, UBE2C gene, CKS2 gene, MKI67 gene, FOXM1 gene, UBE2T gene, MCM4 gene, CKAP2 gene, HN1 gene, KPNA2 gene, H2AFX gene, H2AFZ gene, CDK1 gene, PTTG1 gene, CDC20 gene, MYBL2 gene and RRM2 gene.

The measurement of gene expression can be carried out by the measurement of a transcript. Hereinafter, the method for measuring a gene transcript will be specifically described. The method for measuring a gene transcript is known in the art. The method will be described below with reference to or by citation of the description about a method for measuring a gene transcript or a translation product in Japanese Patent Laid-Open No. 2016-13081. Also, a typical method for measuring a gene transcript will be described below. However, the method is not limited thereto, and a measurement method known in the art can be used.

The measurement of a transcript of the identification gene may be the measurement of a mRNA level or may be the measurement of a cDNA level obtained by reverse transcription from mRNA. In general, the measurement of a gene transcript adopts a method of measuring the expression level of the gene as an absolute value or a relative value using a nucleotide primer or probe comprising the whole or a portion of the nucleotide sequence of the gene.

The measurement of a gene transcript can be a nucleic acid detection and/or quantification method known in the art and is not particularly limited. Examples thereof include hybridization techniques, nucleic acid amplification techniques and RNA sequencing (RNA-Seq) analysis techniques.

The "hybridization technique" is a method of using, as a probe, a nucleic acid fragment having a nucleotide sequence complementary to the whole or a portion of the nucleotide sequence of a target nucleic acid to be detected, and detecting or quantifying the target nucleic acid or a fragment thereof through the use of the base pairing between the nucleic acid and the probe. In this aspect, the target nucleic acid corresponds to mRNA or cDNA of each gene constituting an identification marker, or a fragment thereof. In general, the hybridization technique is preferably performed under stringent conditions in order to eliminate unintended nonspecifically hybridizing nucleic acids. The highly stringent conditions involving a low salt concentration and a high temperature as mentioned above is more preferred. Some methods that differ in detection approach are known as the hybridization technique. For example, Northern blot (Northern hybridization technique), a microarray technique, a surface plasmon resonance technique or a quartz crystal microbalance technique is suitable.

The "Northern blot" is the most general method for analyzing gene expression and is a method of separating total RNA or mRNA prepared from a sample by electrophoresis using agarose gel or polyacrylamide gel, etc. under denaturation conditions, and transferring (blotting) the product to a filter, followed by the detection of a target nucleic acid using a probe having a nucleotide sequence specific for the target RNA. The probe may be labeled with an appropriate marker such as a fluorescent dye or a radioisotope and thereby enables the target nucleic acid to be quantified using a measurement apparatus, for example, a chemiluminescence photographing and analysis apparatus (e.g., Light Capture; ATTO Corp.), a scintillation counter, or an imaging analyzer (e.g., FUJIFILM Corp.: BAS series). The Northern blot is a technique well known and prominent in the art. See, for example, Greene, M.R. and Sambrook, J. (2012) mentioned above.

The "microarray technique" is a method of arranging, on a substrate, small spots at a high density of a probe which is a nucleic acid fragment complementary to the whole or a portion of the nucleotide sequence of a target nucleic acid, reacting the resulting solid-phase microarray or microchip with a sample containing the target nucleic acid, and detecting a nucleic acid hybridized with the substrate spot through fluorescence or the like. The target nucleic acid may be RNA such as mRNA, or DNA such as cDNA. The detection or quantification can be achieved by detecting or measuring fluorescence or the like based on the hybridization of the target nucleic acid, etc. using a microplate reader or a scanner. The mRNA level or the cDNA level or an abundance ratio thereof to reference mRNA can be determined from the measured fluorescence intensity. The microarray technique is also a technique well known in the art. See, for example, a DNA microarray technique (DNA Microarray and Latest PCR Method (2000), Masaaki Muramatsu and Hiroyuki Nawa ed., Gakken Medical Shujunsha Co., Ltd.).

The "surface plasmon resonance (SPR) technique" is a method of very highly sensitively detecting or quantifying an adsorbed matter on the surface of a thin metal film through the use of a surface plasmon resonance phenomenon in which, as the incident angle of laser beam used to irradiate the thin metal film is changed, reflexed light intensity attenuates markedly at a particular incident angle (resonance angle). In the present invention, for example, a probe having a sequence complementary to the nucleotide sequence of a target nucleic acid is immobilized on the surface of the thin metal film, and the other surface portion of the thin metal film is subjected to blocking treatment. Then, a sample collected from a test subject or a healthy subject or a healthy subject group is distributed to the surface of the thin metal film so that the target nucleic acid and the probe form base pairing. The target nucleic acid can be detected or quantified from the difference in measurement value between before and after the samples distribution. The detection or quantification by the surface plasmon resonance technique can be performed using, for example, an SPR sensor commercially available from Biacore/Cytiva. This technique is well known in the art. See, for example, Kazuhiro Nagata and Hiroshi Handa, Real-Time Analysis of Biomolecular Interactions, Springer-Verlag Tokyo, Tokyo, Japan, 2000.

The "quartz crystal microbalance (QCM) technique" is mass spectrometry of quantitatively determining a very small amount of an adsorbed matter from the amount of change in resonance frequency through the use of a phenomenon in which, when a substance is adsorbed to the surface of an electrode attached to a quartz crystal unit, the resonance frequency of the quartz crystal unit is decreased according to the mass thereof. The detection or quantification by this method can also employ a commercially available QCM sensor, as in the SPR technique. For example, a probe having a sequence complementary to the nucleotide sequence of a target nucleic acid is immobilized on the surface of the electrode, and the target nucleic acid can be detected or quantified from the base pairing between the probe and the target nucleic acid in a sample collected from a test subject or a healthy subject or a healthy subject group. This technique is well known in the art. See, for example, Christopher J. et al., 2005, Self-Assembled Monolayers of a Form of Nanotechnology, Chemical Review, 105: 1103-1169 and Toyosaka Moriizumi and Takamichi Nakamoto, (1997) Sensor Engineering, Shokodo Co., Ltd.

The "nucleic acid amplification technique" is a method of amplifying a particular region of a target nucleic acid with nucleic acid polymerase using forward and reverse primers. Examples thereof include PCR (including RT-PCR), NASBA, ICAN, and LAMP(R) (including RT-LAMP). PCR is preferred. The method for measuring a gene transcript by use of the nucleic acid amplification technique employs a quantitative nucleic acid amplification technique such as real-time RT-PCR. Further, an intercalator technique using SYBR(R) Green or the like, a Taqman(R) probe technique, digital PCR, and a cycling probe technique are known as real-time RT-PCR, and any of the methods can be used. All of these methods are known in the art and are described in appropriate protocols in the art. See such protocols.

The "RNA sequencing (RNA-Seq) analysis technique" refers to a method of converting RNA to cDNA through reverse transcription reaction, and counting the number of reads thereof using a next-generation sequencer (e.g., which includes, but not limited to, HiSeq series (Illumina, Inc.) and Ion Proton System (Thermo Fisher Scientific Inc.)) to measure the expression quantity of the gene. All of these methods are known in the art and are described in appropriate protocols in the art. See such protocols.

Hereinafter, the method for quantifying a gene transcript by RT-PCR will be briefly described by taking one example. The real-time RT-PCR is a nucleic acid quantification method of performing PCR using a temperature cycler apparatus having a function of detecting fluorescence intensity derived from an amplification product in a reaction system in which cDNA prepared through reverse transcription reaction from mRNA in a sample is used as a template and the PCR amplification product is specifically fluorescently labeled. The amount of the amplification product from a target nucleic acid is monitored in real time during reaction, and the results are subjected to regression analysis in a computer. The method for labeling the amplification product includes a method using a fluorescently labeled probe (e.g., TaqMan(R) PCR) and an intercalator method using a reagent specifically binding to double-stranded DNA. The TaqMan(R) PCR employs a probe modified at its 5'-terminal portion with a quencher material and at its 3'-terminal portion with a fluorescent dye. The quencher material at the 5'-terminal portion usually inhibits the fluorescent dye at the 3'-terminal portion. Upon PCR, the probe is degraded by the 5'→3' exonuclease activity of Taq polymerase, thereby canceling the inhibition of the quencher material. Therefore, fluorescence is emitted. The amount of the fluorescence reflects the amount of the amplification product. When the amplification product reaches a detection limit, the number of cycles (CT) is in inverse correlation with the initial amount of the template. Therefore, in the real-time measurement technique, the initial amount of the template is quantified by measuring CT. Provided that CT is measured using several known amounts of the template to prepare a calibration curve, the absolute value of the initial amount of the template in an unknown sample can be calculated. For example, M-MLV RTase, ExScript RTase (Takara Bio Inc.), or Super Script II RT (Thermo Fisher Scientific Inc.) can be used as reverse transcriptase for use in RT-PCR.

The reaction conditions of real-time PCR are generally based on PCR known in the art and vary depending on the base length of a nucleic acid fragment to be amplified and the amount of a templated nucleic acid as well as the base length and Tm value of the primer used, the optimum reaction temperature and optimum pH of the nucleic acid polymerase used, etc. Therefore, the reaction conditions can be appropriately determined according to these conditions. As one example, usually, denaturation reaction is performed at 94 to 95°C for 5 seconds to 5 minutes; annealing reaction is performed at 50 to 70°C for 10 seconds to 1 minute; and elongation reaction is performed at 68 to 72°C for 30 seconds to 3 minutes. This cycle can be repetitively performed 15 to 40 times to perform the elongation reaction. In the case of using a kit commercially available from any of the manufacturers, this operation can be performed according to a protocol attached to the kit, as a rule.

The nucleic acid polymerase for use in real-time PCR is DNA polymerase, particularly, thermostable DNA polymerase. Such nucleic acid polymerase is commercially available as various types, which may be used. Examples thereof include Taq DNA polymerase attached to Applied Biosystems TaqMan MicroRNA Assays Kit (Thermo Fisher Scientific Inc.) described above. Particularly, such a commercially available kit is useful because a buffer or the like optimized for the activity of the attached DNA polymerase is attached to the kit.

The gene expression analysis method of the present invention comprises, in addition to the step (a), a step of (b) measuring at least one internal standard gene selected from the group consisting of ABCF3, FBXW5, MLLT1, FAM234A, PITPNM1, WDR1, NDUFS7, and AP2A1.

The step (a) and the step (b) can be performed at the same time or may be separately performed such that either of these steps may be performed first. In a preferred embodiment, the steps (a) and (b) are performed at the same time.

The gene expression analysis method of the present invention comprises, after the step (a) and the step (b), a step of (c) normalizing the expression level of the desired gene using the expression level of the internal standard gene.

In the present specification, the term "normalizing" refers to enabling the expression level of the desired gene measured under particular conditions in a test sample to be compared with the expression level of the desired gene measured under different conditions in the test sample. More specifically, the term means that the expression level of the desired gene measured under particular conditions in a test sample is compared with the expression level of the internal standard gene measured under the same conditions in the test sample, thereby calculating the expression level of the desired gene in the test sample as a relative value to the expression level of the internal standard gene.

In the normalization step, the method for calculating the expression level of the desired gene as a relative value is not limited as long as this expression level can be compared with the expression level of the desired gene measured under different conditions in the test sample. The expression level of the desired gene measured under particular conditions in a test sample can be indicated as a relative value, for example, by dividing its value by the value of the expression level of the internal standard gene measured under the same conditions in the test sample.

The expression level of the desired gene normalized by the step (c) can be relatively compared with the expression level of the gene measured under different conditions and normalized by a similar approach.

### [Examples]

### (Example 1. RNA preparation)

Total RNA was extracted from surgically collected breast cancer tissues using ISOGEN (Nippon Gene Co., Ltd., Tokyo, Japan). Normal mammary gland tissues and some breast cancer tissues were purchased from an overseas agency, and total RNA was extracted therefrom in the same manner as above. Samples from which 125 µg or more of total RNA was able to be obtained were subsequently subjected to poly(A) + RNA purification using MicroPoly(A) purist Kit (Ambion, Austin, TX, USA).

The human common reference RNA used was Human Universal Reference RNA Type I (MicroDiagnostic, Tokyo, Japan) or Human Universal Reference RNA Type II (MicroDiagnostic).

### (Example 2. Exhaustive gene expression analysis)

The DNA microarray used in the obtainment of gene expression profiles using poly(A) + RNA (designated as "system 1") was a glass slide on which 31,797 types of synthetic DNAs (80 mers) (MicroDiagnostic) corresponding to human-derived transcripts were arrayed using a custom arrayer. On the other hand, the DNA microarray used for the obtainment of gene expression profiles using total RNA (designated as "system 2") was a glass slide on which 14,400 types of synthetic DNAs (80 mers) (MicroDiagnostic) corresponding to human-derived transcripts were arrayed using a custom arrayer.

As for the specimen-derived RNA, labeled cDNA was synthesized from 2 µg of poly(A) + RNA for the system 1 and from 5 µg of total RNA for the system 2 using SuperScript II (Invitrogen Life Technologies, Carlsbad, CA, USA) and Cyanine 5-dUTP (Perkin-Elmer Inc.). Likewise, for the human common reference RNA, labeled cDNA was synthesized from 2 µg of poly(A) + RNA or 5 µg of total RNA using SuperScript II and Cyanine 3-dUTP (Perkin-Elmer Inc.).

Hybridization to the DNA microarray was performed using Labeling and Hybridization kit (MicroDiagnostic).

Fluorescence intensity after hybridization to the DNA microarray was measured using GenePix 4000B Scanner (Axon Instruments, Inc., Union city, CA, USA). An expression ratio was calculated by dividing the fluorescence intensity of the specimen-derived Cyanine-5-labeled cDNA by the fluorescence intensity of the human common reference-derived Cyanine-3-labeled cDNA (fluorescence intensity of the specimen-derived Cyanine-5-labeled cDNA / fluorescence intensity of the human common reference-derived Cyanine-3-labeled cDNA). Further, normalization was performed by multiplying the calculated expression ratio by a normalization factor using GenePix Pro 3.0 software (Axon Instruments, Inc.,). Next, the expression ratio was converted to log 2, and the converted value was designated as a log 2 ratio. The conversion of the expression ratio was performed using Excel software (Microsoft, Bellevue, WA, USA) and MDI gene expression analysis software package (MicroDiagnostic).

### (Example 3. Internal standard gene useful in identifying breast cancer subtype)

In this Example, genes whose expression pattern did not vary depending on any subtype of breast cancer were determined.

According to the RNA preparation method and the exhaustive gene expression analysis method described above in Examples 1 and 2, gene expression profiles of 14,400 genes were obtained as to each of specimens of 470 cases in total involving breast cancer tissues (453 cases) and normal mammary gland tissues (17 cases).

Among the obtained gene expression profiles, genes were picked up which substantially rarely varied in expression ratio depending on any subtype of breast cancer. Specifically, internal standard genes were selected from genes for which the number of specimens having no detectable signal was 3 or less; the absolute value of the expression ratio was less than 0.45; the standard deviation was less than 0.35; the value of maximum value - minimum value was less than 2.2; and the average value of "sum of medians" exceeded 400. As a result, eight genes, ABCF3, FBXW5, MLLT1, FAM234A, PITPNM1, WDR1, NDUFS7, and AP2A1, were successfully selected as internal standard genes.

The following table shows standard deviations, maximum values, and minimum values in the gene expression profiles of ABCF3, FBXW5, MLLT1, FAM234A, PITPNM1, WDR1, NDUFS7, and AP2A1.

**[Table 3]**

| Symbol | ABCF3 | FBXW5 | MLLT1 | FAM234A PITPNM1 | WDR1 | NDUFS7 | AP2A1 |
|---|---|---|---|---|---|---|---|
| Standard deviation | 0.253 | 0.247 | 0.292 | 0.296 0.269 | 0.329 | 0.273 | 0.282 |
| Maximum value | 1.647 | 1.213 | 1.052 | 1.240 1.132 | 0.739 | 0.637 | 0.602 |
| Minimum value | -0.425 | -0.951 | -1.132 | -0.501 -1.053 | -2.250 | -1.305 | -1.232 |

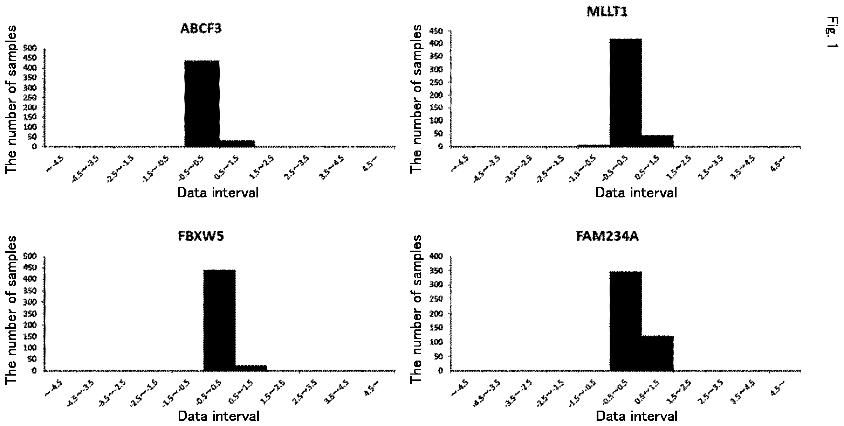

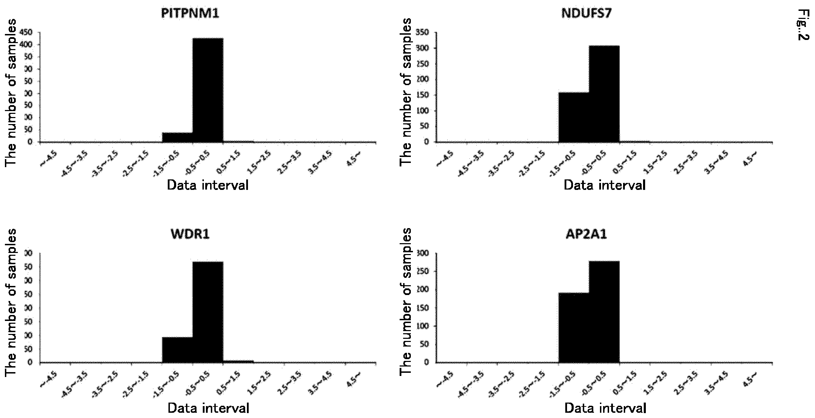

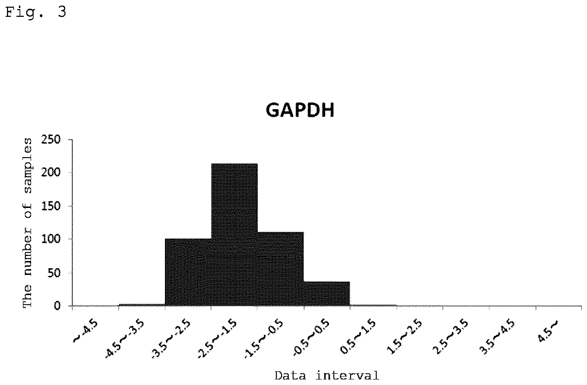

Table 4 given below and Figures 1 to 3 show a distribution at each data interval of the expression ratio delimited by 1.0 in the gene expression profiles of the eight internal standard genes. Table 4 and Figures 1 to 3 also show a distribution of GAPDH (Accession No: NM_002046) heretofore used as a housekeeping gene for the comparison of the eight internal standard genes.

**[Table 4]**

| Data interval | Frequency | | | | | | | | |
|---|---|---|---|---|---|---|---|---|---|
| | ABCF3 | FBXW5 | MLLT1 | FAM234A | PITPNM1 | WDR1 | NDUFS7 | AP2A1 | GAPDH |
| ~-4.5 | 0 | 0 | 0 | 0 | 0 | 0 | 0 | 0 | 1 |
| ~4.5~-3.5 | 0 | 0 | 0 | 0 | 0 | 0 | 0 | 0 | 3 |
| -3.5~-2.5 | 0 | 0 | 0 | 0 | 0 | 0 | 0 | 0 | 101 |
| -2.5~-1.5 | 0 | 0 | 0 | 0 | 0 | 1 | 0 | 0 | 214 |
| -1.5~-0.5 | 0 | 4 | 7 | 1 | 39 | 93 | 159 | 191 | 111 |
| -0.5~0.5 | 437 | 442 | 419 | 347 | 427 | 368 | 308 | 278 | 37 |
| 0.5~1.5 | 32 | 24 | 44 | 122 | 4 | 8 | 3 | 1 | 2 |
| 1.5~2.5 | 1 | 0 | 0 | 0 | 0 | 0 | 0 | 0 | 1 |
| 2.5~3.5 | 0 | 0 | 0 | 0 | 0 | 0 | 0 | 0 | 0 |
| 3.5~4.5 | 0 | 0 | 0 | 0 | 0 | 0 | 0 | 0 | 0 |
| 4.5~ | 0 | 0 | 0 | 0 | 0 | 0 | 0 | 0 | 0 |

As shown in Table 4 and Figures 1 to 3, the eight internal standard genes less varied in expression ratio among samples than the GAPDH gene. Most of distributions of the expression ratio fell within the median data interval from -0.5 to 0.5.

### (Example 4. Identification of breast cancer subtype by gene expression analysis)

The present inventors successfully determined a gene group that exhibited an expression pattern characteristic of squamous cell cancer (hereinafter, referred to as "a group"), a gene group that exhibited an expression pattern characteristic of phyllodes tumor (hereinafter, referred to as "b group"), a gene group that exhibited an expression pattern characteristic of cancer (hereinafter, referred to as "c group"), a gene group that exhibited an expression pattern characteristic of normal tissues (hereinafter, referred to as "d group"), a gene group that exhibited an expression pattern characteristic of normal-like cases (hereinafter, referred to as "e group"), a gene (hereinafter, referred to as "TNBC1") group that exhibited an expression pattern characteristic of triple negative groups and exhibited an expression pattern characteristic of normal tissues or normal-like cases (hereinafter, referred to as "f group"), a gene (hereinafter, referred to as "TNBC2") group that exhibited an expression pattern characteristic of triple negative cases (hereinafter, referred to as "g group"), a gene (hereinafter, referred to as "TNBC3") group that exhibited an expression pattern characteristic of triple negative cases and exhibited an expression pattern also similar to that of a gene defining poorly characterized cancer (indeterminable one) (hereinafter, referred to as "h group"), a gene group that exhibited an expression pattern characteristic of HER2+-like cases (hereinafter, referred to as "i group"), a gene (hereinafter, referred to as HER2 amplification-1") group that was associated with HER2 amplification and resided chromosomally at a position near the HER2 gene (hereinafter, referred to as "j group"), a gene (hereinafter, referred to as HER2 amplification-2") group that was associated with HER2 amplification and was other than the j group (hereinafter, referred to as "k group"), a hormone sensitivity-related gene group (hereinafter, referred to as "1 group"), ESR1 (hereinafter, referred to as "m group"), a differentiation-related gene group (hereinafter, referred to as "n group") and a cell cycle-related gene group (hereinafter, referred to as "o group") from the gene expression profiles obtained in Example 3. 199 genes were determined as genes contained in these a to o groups (identification marker gene sets for identifying a subtype of breast cancer). The following tables show the genes contained in the identification marker gene sets.

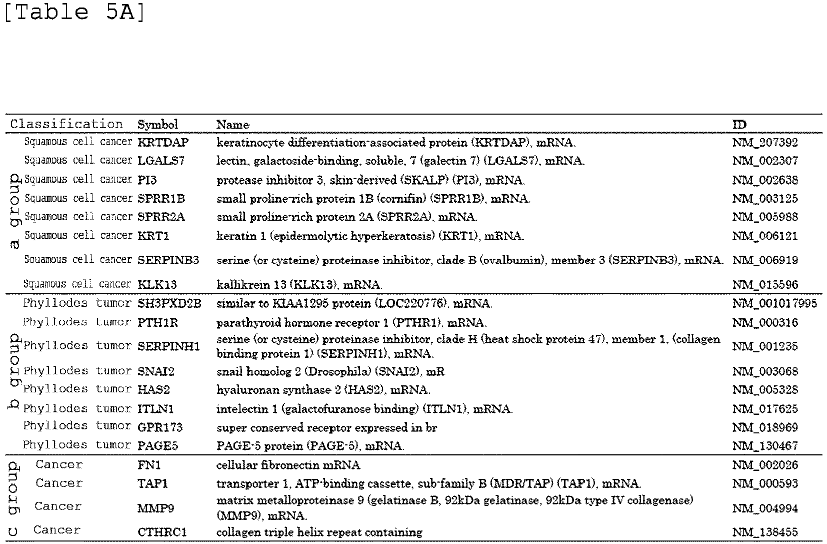

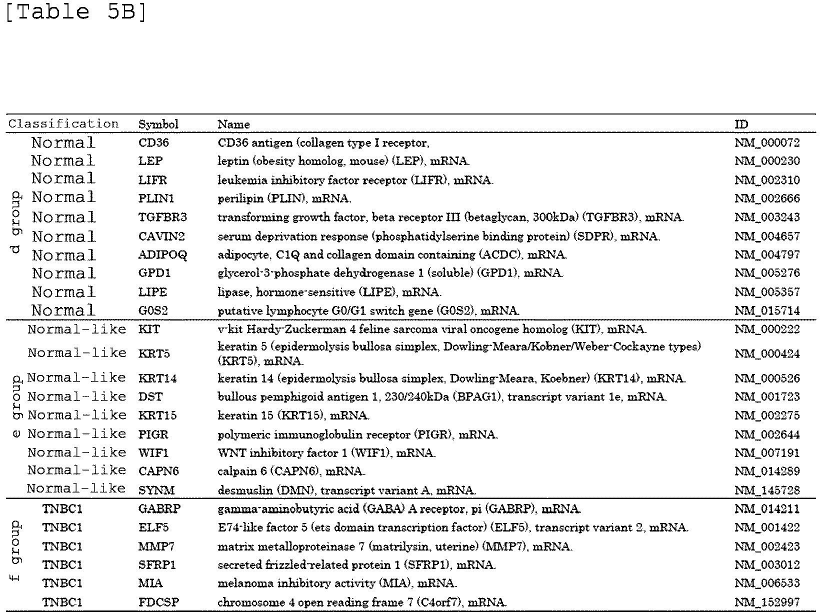

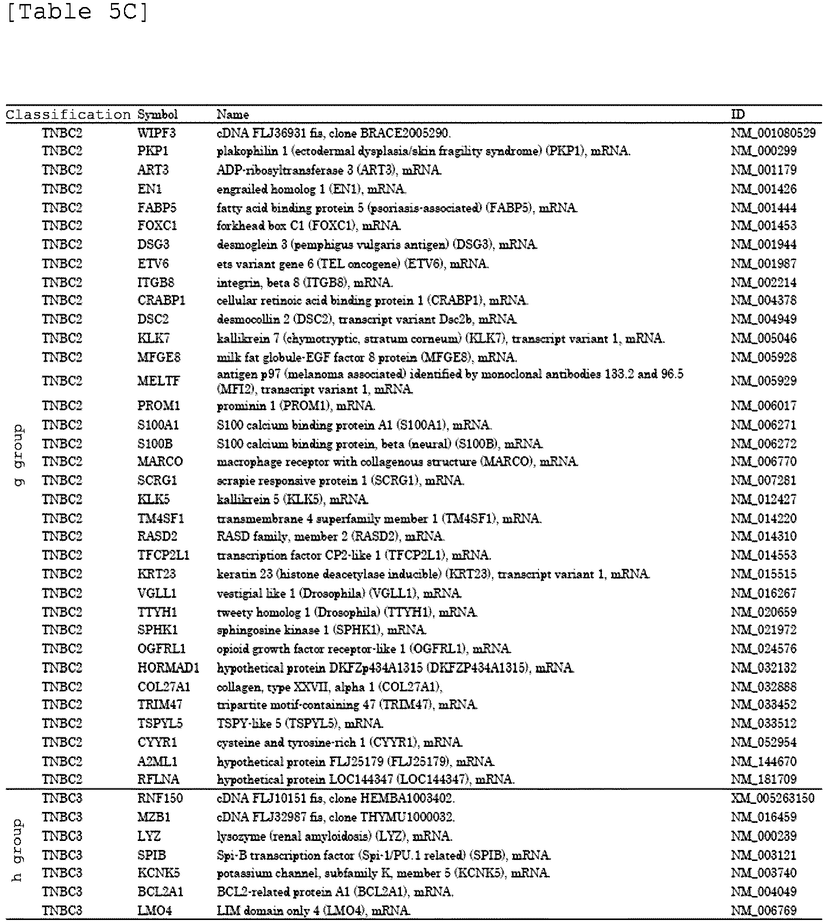

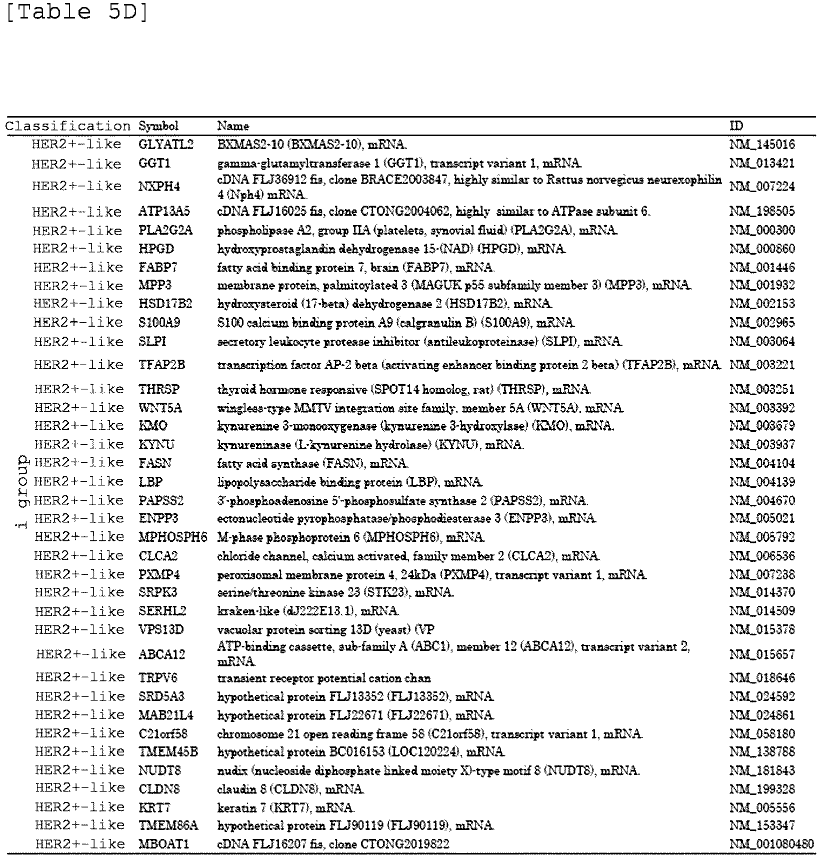

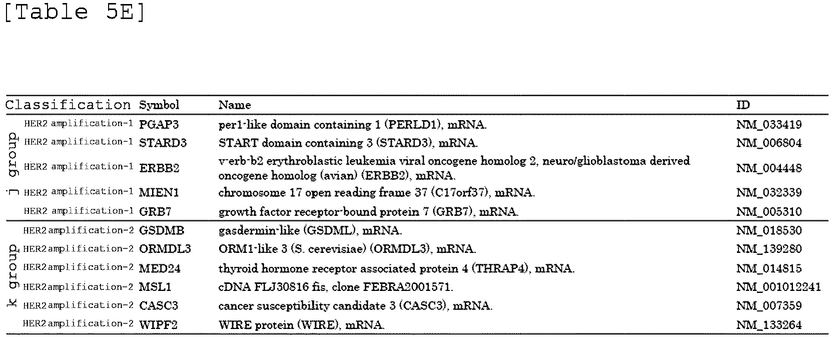

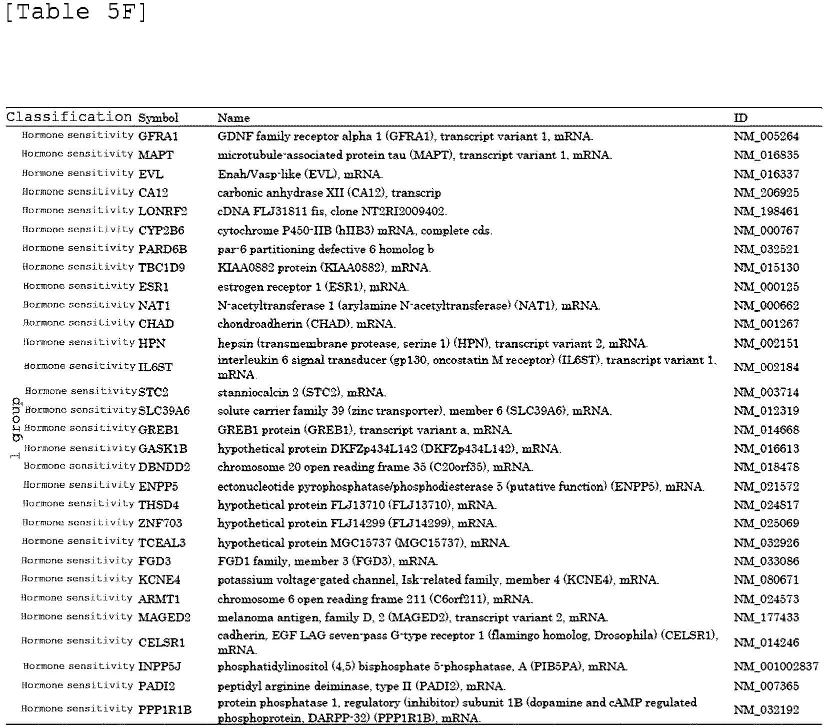

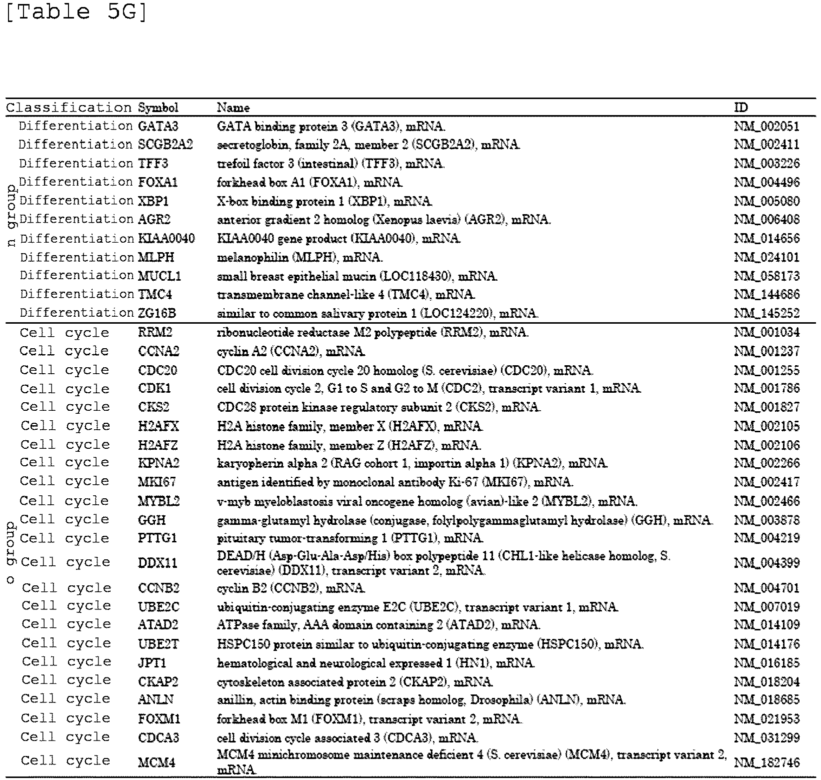

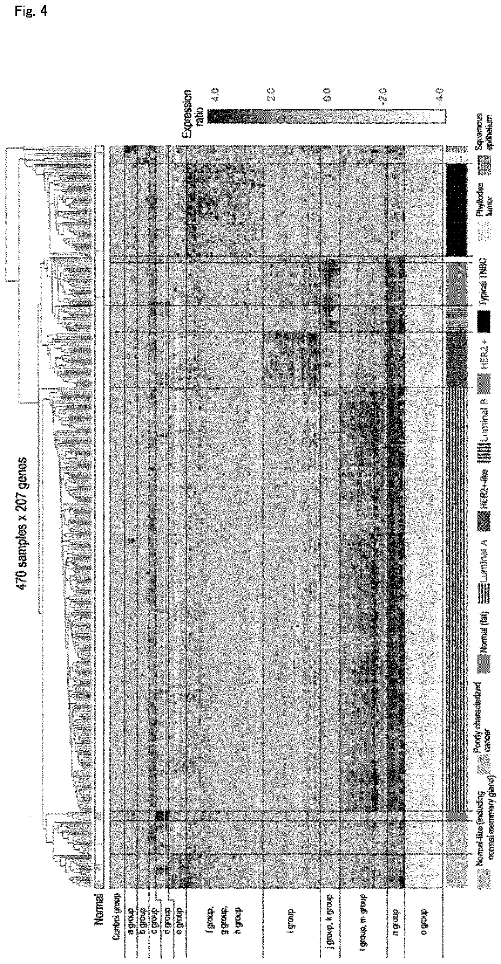

The gene expression level of each gene was measured (data not shown) as to 207 genes in total involving the eight internal standard genes and the identification marker gene sets consisting of the 199 genes, followed by cluster analysis. The cluster analysis was conducted by a gene averaging technique based on a Euclidean distance using ExpressionView Pro software (MicroDiagnostic). The results of the cluster analysis are shown in Figure 4. As shown in Figure 4, as a result of conducting hierarchical cluster analysis on the basis of the expression profiles of the 207 extracted genes, the genes contained in the a to o groups exhibited an expression ratio characteristic of each subtype, whereas no variation in expression was observed in the genes of a control group for any subtype. By the hierarchical cluster analysis, the subtypes of breast cancer were able to be classified into clusters of a normal-like group, an indeterminable group, a normal group, a luminal A group, a HER2+-like group, a luminal B group, a HER2+ group, a triple negative group, and other groups.

The following tables show sequence information on the probes against the 207 genes used in Examples 3 and 4.

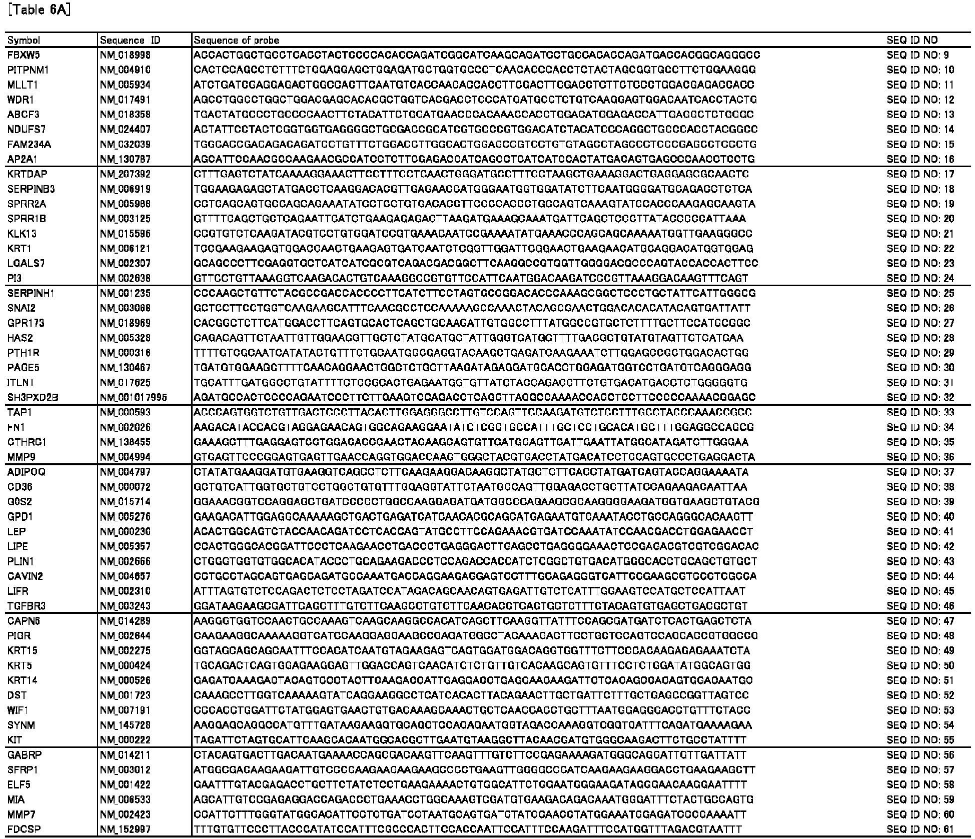

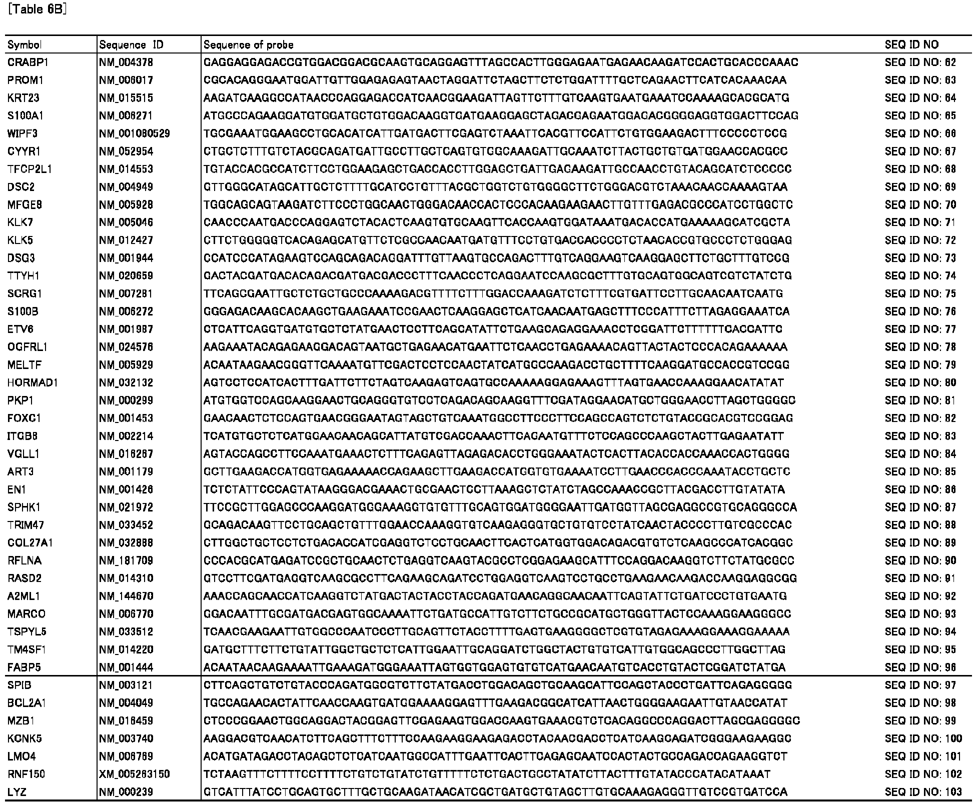

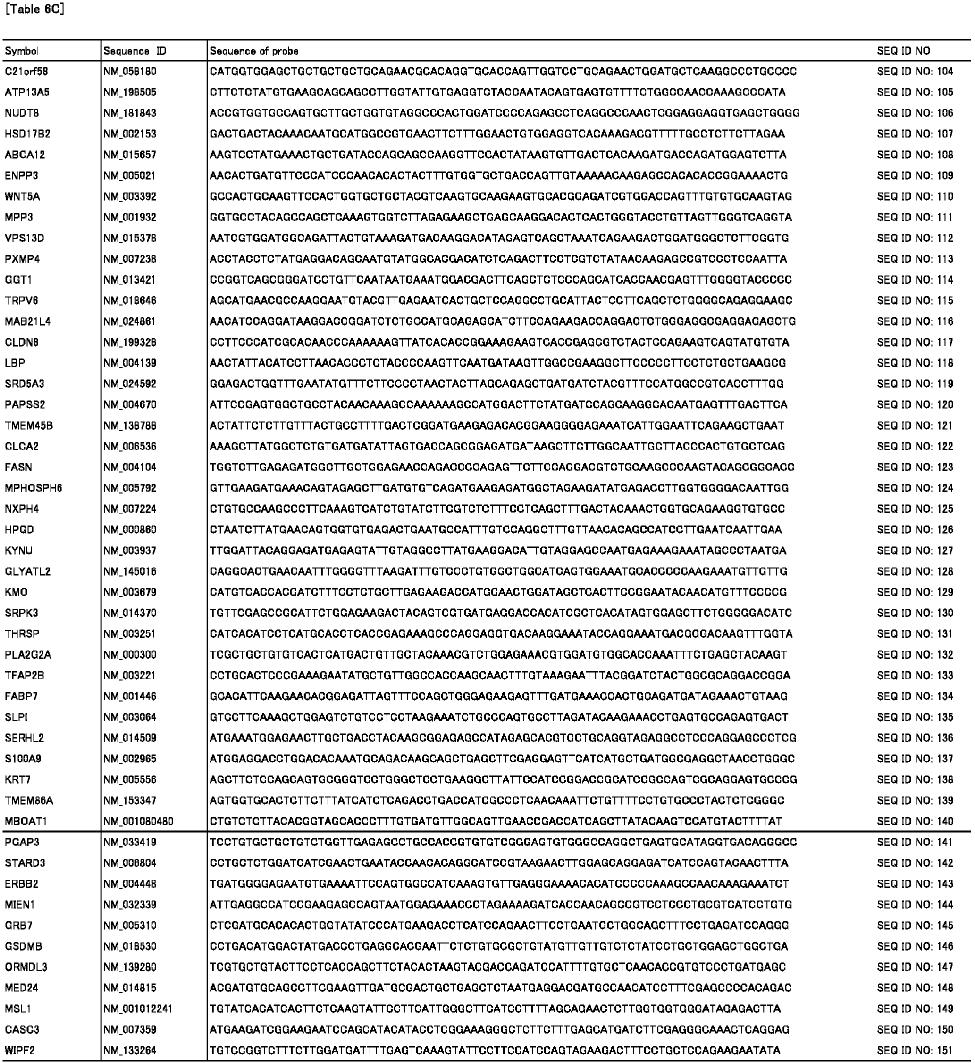

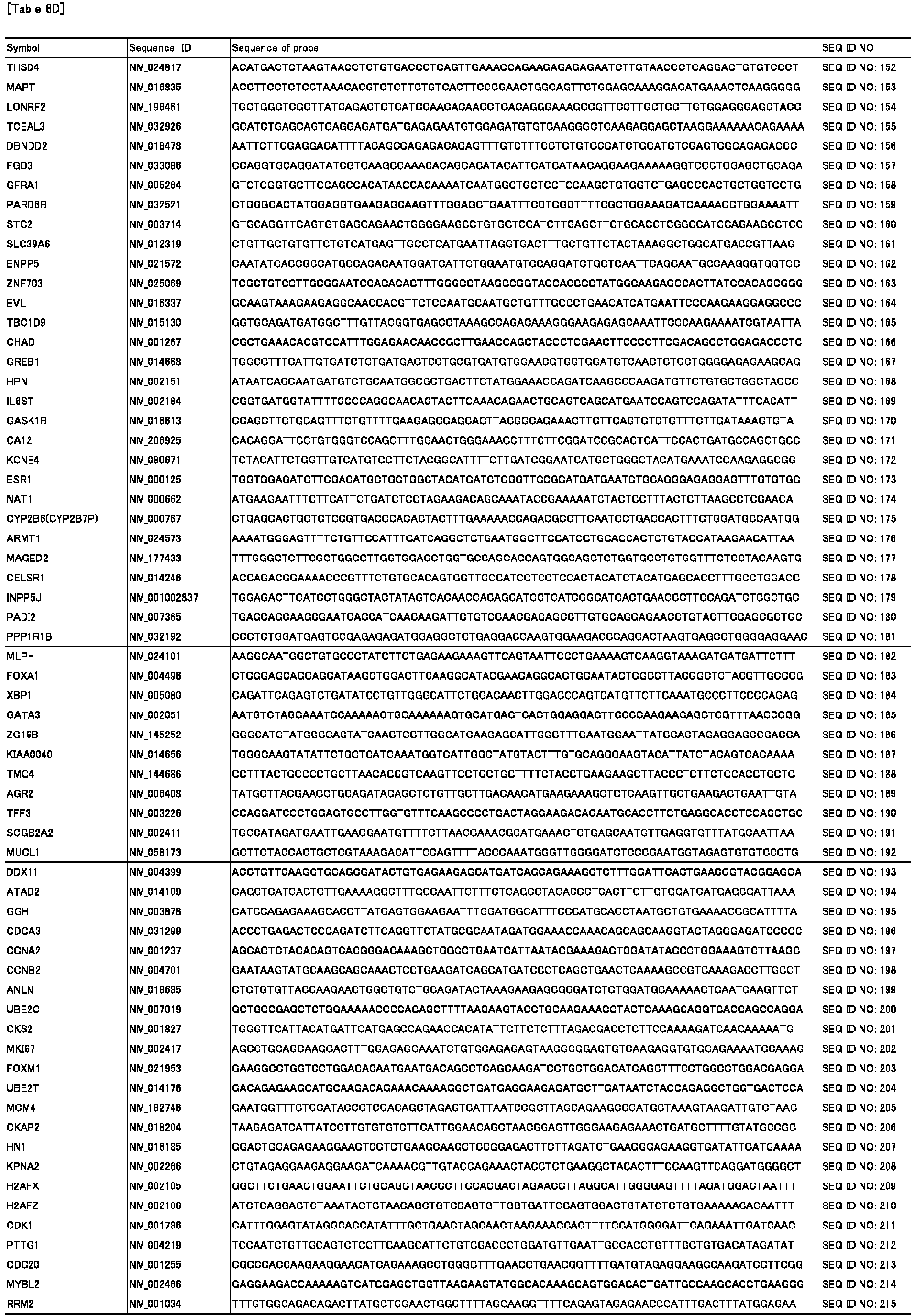

## Claims

1. A gene expression analysis method for a test sample, comprising the steps of:
(a) measuring an expression level of a desired gene;
(b) measuring an expression level of at least one internal standard gene selected from the group consisting of ABCF3, FBXW5, MLLT1, FAM234A, PITPNM1, WDR1, NDUFS7, and AP2A1; and
(c) normalizing the expression level of the desired gene using the expression level of the internal standard gene.

2. The gene expression analysis method according to claim 1, wherein
the test sample is a sample derived from a breast cancer patient.

3. The gene expression analysis method according to claim 2, wherein
the desired gene is a gene for identifying or classifying a subtype of breast cancer.

4. An internal standard gene for gene expression analysis consisting of at least one gene selected from the group consisting of FBXW5, PITPNM1, MLLT1, WDR1, ABCF3, NDUFS7, FAM234A, and AP2A1.

5. The internal standard gene according to claim 4, wherein
the internal standard gene is used in gene expression analysis for a test sample derived from a breast cancer patient.

6. The internal standard gene according to claim 5, wherein
the gene expression analysis for the test sample derived from a breast cancer patient is gene expression analysis for identifying a subtype of breast cancer.

7. A composition for expression analysis of an internal standard gene, comprising a unit for measuring an expression level of the internal standard gene for gene expression analysis consisting of at least one gene selected from the group consisting of FBXW5, PITPNM1, MLLT1, WDR1, ABCF3, NDUFS7, FAM234A, and AP2A1.

8. The composition for expression analysis of an internal standard gene according to claim 7 for identifying or classifying breast cancer, wherein the internal standard gene is used in gene expression analysis for a test sample derived from a breast cancer patient.

9. The composition for expression analysis of an internal standard gene according to claim 8 for identifying or classifying breast cancer, wherein the gene expression analysis for a test sample derived from a breast cancer patient is gene expression analysis for identifying a subtype of breast cancer.

10. The composition for expression analysis of an internal standard gene for identifying or classifying breast cancer according to claim 8, wherein
the unit for measuring an expression level of the gene is at least one unit selected from the group consisting of a primer and a probe against the gene, and labeled forms thereof.

11. The composition for expression analysis of an internal standard gene for identifying or classifying breast cancer according to claim 9, wherein
the unit for measuring an expression level of the gene is at least one unit selected from the group consisting of a primer and a probe against the gene, and labeled forms thereof.

12. The composition for expression analysis of an internal standard gene for identifying or classifying breast cancer according to claim 8, wherein
the composition is intended for PCR, a microarray, or RNA sequencing.

13. The composition for expression analysis of an internal standard gene for identifying or classifying breast cancer according to claim 9, wherein
the composition is intended for PCR, a microarray, or RNA sequencing.


### [Sequence Listing]

SEQUENCE LISTING

<110>  Fukushima Medical University

<120>  Internal control genes

<130>

<160>  215

<170>  PatentIn version 3.5

<210>  1
<211>  2362
<212>  DNA
<213>  Homo sapiens

<400>  1
ctcggccatc gatgcctcgg ggctcgtggc gtcgggcctg gcgggctccc cgttgggcgg      60

gggcggcggt ccgagcggct gcggcagcgg aagcgggtcc gggtcgcgca gggcgggggc     120

ggcgcagcgg gcggaggcgg ccgtgggccg cctgggcaga atgtcacgat ggacgagggc     180

ggcacgcccc tgctccccga cagcctggtc taccagatct tcctgagcct gggcccggcc     240

gacgtgctgg ccgccgggct ggtgtgccgc caatggcagg ccgtgtcgcg ggacgagttc     300

ctgtggaggg agcagttcta ccgctactac caggtggccc gcgacgtgcc ccgacaccca     360

gcggccatgt cctggtacga ggagttccag cggctgtatg acacggtgcc ctgcgtggag     420

gtgcagacgc tgcgggaaca cacagaccag gtcctgcacc tcagcttctc ccattccggg     480

taccagttcg cgtcctgctc caaggactgc actgtgaaga tctggagcaa cgacctgacc     540

atctcgctgc tgcacagcgc ggacatgcgg ccctacaact ggagctacac ccagttctcc     600

cagttcaaca aggacgactc gctactgctg gcctcggggg tgttcctggg gccgcacaac     660

tcctcatccg gcgagattgc tgtcatcagc ctagactcct tcgcgctgct gtcccgcgtg     720

cggaacaagc cctatgacgt gtttggctgt tggctcaccg agaccagcct catctcgggg     780

aacctgcacc gcatcggaga tatcacctcc tgctcggtgc tgtggctcaa caatgccttc     840

caggatgtgg agtcagagaa cgtcaacgtg gtgaagcggc tgttcaagat ccagaacctc     900

aatgccagca ccgtccgcac ggtgatggtg gccgactgca gccgcttcga cagccctgac     960

ctgctgctgg aagccggtga cccggccacg tccccctgcc gcatctttga cctgggcagc    1020

gacaacgagg aggtggtggc tggcccggcc cccgcccacg ccaaggaggg cttgcggcac    1080

tttctggacc gcgtgctgga ggggcgggcg cagccacagc tgtcggagcg catgctagag    1140

accaaggtgg ccgagctgct ggcccagggc cacaccaagc cacccgagcg cagtgccaca    1200

ggcgccaaga gcaagtacct catcttcacc actggctgcc tcacctactc cccacaccag    1260

atcggcatca agcagatcct gccacaccag atgaccacgg cagggcccgt gctgggtgag    1320

ggccggggct ccgatgcctt cttcgacgcg ctggaccacg tcatagacat acacggacac    1380

atcatcggca tgggcctgtc gcccgacaac aggtacctgt acgtgaacag ccgcgcctgg 1440

cccaacggtg cggtggtggc cgaccccatg cagccgccac caatcgcgga ggagattgac 1500

ctgctggtgt tcgacctcaa gaccatgcgg gaggtgaggc gggctctgcg tgcgcaccgc 1560

gcctacacgc ccaacgacga gtgcttcttc atcttcctgg acgtcagcag ggacttcgtg 1620

gccagcgggg cggaggaccg gcacggctac atctgggacc gccactacaa catctgtctg 1680

gccaggctgc ggcacgagga tgtggtcaac tcagtggtct tcagtcccca ggagcaggag 1740

ctgctgctca cggccagcga cgacgccacc atcaaagcct ggcgctcccc acgcaccatg 1800

cgcgtcctcc aggcacctcg cccacggcct cgcaccttct tctcctggct tgccagccag 1860

aggcgctgag gtgtgctggg tgcactggag ccaccgggac cccttgagga catcgccagg 1920

ctctgtggct ttttcccgag cgggagaggt ggagatgctt atagcagtta cgccttagga 1980

aggggacaac caggccccgc cacacgctca cacacaaacc tgctcacgca gctgtgatgc 2040

ttggcacggg gtggccagtg cagatggagc ccaaggcccc ctcggcctcc tgggcaactt 2100

ggggtacaca ggatactggg ggtgccgctc ctcactcaac cccaggctag gggtacacct 2160

gacccagctg gcctcggccc ggggcacctt cggctggtcc tgtggggccc tggacggtgg 2220

cccagtggtg gcaggggctg ctcctggctg tggttgtgcg cccggggctt gggagcggcc 2280

ggtcacgctg ctgtgggccc gagtgtgttg catgtccacg caccacccgt tcagggccct 2340

gaataaacag ttggcaacag ca 2362

<210> 2
<211> 4217
<212> DNA
<213> Homo sapiens

<400> 2
aggggcgccg gcgggcggat cgagaggaag gtcggggcca tgggccggca ctgcgcctcg 60

ggagggtccg gccaccgctg gaacccgagg ccggggctgg gggcgctccg ggctccgacc 120

cacgggccgg ccggccctgc ccgggctggg tgaggggcgc ccgcctcaag ctagaggagg 180

agcggaggcc gcgcgcggcc cgccgagcgc cttcaggatg ctcatcaagg aataccacat 240

tctgctgccc atgagcctgg acgagtacca ggtggcccag ctctacatga tccagaaaaa 300

gagccgggag gagtctagtg gtgagggcag cggcgtggag atcctggcca accggcccta 360

cacggatggg cccgggggca gcgggcaata cacacacaag gtgtaccacg tgggctccca 420

catcccaggc tggttccggg cactgctgcc caaggctgcc ctgcaggtag aagaggaatc 480

ctggaatgcc tacccctaca cccgaacccg gtacacctgc cctttcgtgg agaaattctc 540

cattgaaatt gagacctatt acctgcctga tggggggcag cagccaaacg tcttcaacct 600

gagcggggcc gagaggagac agcgcatcct ggacaccatc gacatcgtgc gggatgcagt 660

ggccccaggc gagtacaaag cagaagagga cccccggctt tatcactcgg tcaagacggg 720
ccgagggcca ctgtctgatg actgggcacg gacggcggca cagacggggc cccttatgtg 780
tgcctataag ctgtgcaagg ttgagttccg ctactggggc atgcaagcca agatcgagca 840
gttcatccat gatgtaggtc tgcgtcgggt gatgctgcgg gcccaccgcc aggcctggtg 900
ctggcaggat gagtggacag agctgagcat ggctgacatc cgggcactgg aagaggagac 960
tgctcgcatg ctggcccagc gcatggccaa gtgcaacaca ggcagtgagg ggtccgaggc 1020
ccagcccccc gggaaaccga gcaccgaggc ccggtctgcg gccagcaaca ctggcacccc 1080
cgatgggcct gaggcccccc caggcccaga tgcctccccc gatgccagct ttgggaagca 1140
gtggtcctca tcctcccgtt cctcctactc atcccaacat ggaggggctg tgtctcccca 1200
gagcttgtct gagtggcgca tgcagaacat tgcccgagac tctgagaaca gctccgagga 1260
agagttcttt gatgcccacg aaggcttctc ggacagtgag gaggtcttcc ccaaggagat 1320
gaccaagtgg aactccaatg acttcattga tgcctttgcc tccccagtgg aggcagaggg 1380
aacgccagag cctggagccg aggcagctaa aggcattgag gatggggccc aagcacccag 1440
ggactcagag ggcctggatg gagccgggga gctgggggct gaggcatgcg cagtccacgc 1500
cctcttcctt atcctgcaca gcggcaacat cctggactca ggccctggag acgccaactc 1560
caagcaggcg gatgtgcaga cgctgagctc cgccttcgag gccgtcaccc gcatccactt 1620
ccctgaggcc ttgggccacg tggcgctgcg actggtgccc tgtccaccca tctgcgccgc 1680
cgcctatgcc cttgtctcca acctgagccc ttacagccac gatggggaca gcctgtctcg 1740
ctcccaagac cacattccac tggctgccct gccactgctg gccacctcat cctcccgcta 1800
ccagggcgcc gtggccaccg tcattgcccg caccaaccag gcctactcag ccttcctgcg 1860
ctcacctgag ggtgccggct tctgtgggca ggtcgcactg attggagatg gtgttggtgg 1920
catcctgggc tttgatgcac tctgccacag tgctaacgcg ggcaccggga gtcggggcag 1980
cagccgccgt gggagcatga acaatgagct gctctctccg gagtttggcc cagtgcggga 2040
ccccctggca gatggtgtgg aaggcctggg tcggggcagc ccagaaccct cggccttgcc 2100
tccccagcgc atccccagcg acatggccag tcctgagccc gagggctctc agaacagcct 2160
tcaggcagcc cccgcaacca cctcctcctg ggagccccgg cgggcaagca cggccttctg 2220
cccacccgct gccagttccg aggcacctga cggccccagc agcactgccc gccttgactt 2280
caaggtctct ggcttcttcc tcttcggctc cccactgggc ctggtgctgg ctctgcgcaa 2340
aactgtgatg cccgccctgg aggcagccca gatgcgccca gcctgtgaac agatctacaa 2400
cctcttccac gcggctgacc cctgcgcctc acgcctcgag cccctgctgg ccccgaagtt 2460
ccaggccatc gccccactga ccgtgccccg ctaccagaag ttccccctgg gagatggctc 2520

atccctgctg ctggccgaca ctctgcagac gcactccagc ctctttctgg aggagctgga 2580

gatgctggtg ccctcaacac ccacctctac tagcggtgcc ttctggaagg gcagtgagtt 2640

ggccactgac cccccggccc agccagccgc ccccagcacc accagtgagg tggttaagat 2700

cctggagcgc tggtggggga ccaagcggat cgactactcg ctgtactgcc ccgaggcgct 2760

caccgccttt cccaccgtca cgctgcccca cctcttccac gccagctact gggagtccgc 2820

cgacgtggtg gcgttcatcc tgcgccaggt gatcgagaag gagcggccac agctggcgga 2880

atgcgaggag ccgtccatct acagcccggc cttccccagg gagaagtggc agcgaaaacg 2940

cacgcaggtc aagatccgga acgtcacttc caaccaccgg gcgagcgaca cggtggtgtg 3000

cgagggccgc ccccaggtgc taagcgggcg cttcatgtac gggcccctgg acgtcgtcac 3060

gctcactgga gagaaggtgg atgtctacat catgacgcag ccgctgtcgg gcaagtggat 3120

ccactttggc accgaagtca ccaatagctc gggccgcctc accttcccag ttcccccaga 3180

acgcgcgctg ggcattggtg tctaccccgt gcgcatggtg gtcaggggcg accacaccta 3240

tgccgaatgc tgcctgactg tggtggcccg cggcacggag gctgtggtct tcagcatcga 3300

cggctccttc accgccagcg tctccatcat gggcagcgac cccaaggtgc gagctggcgc 3360

cgtggacgtg gtcaggcact ggcaggactc cggctacctg atcgtgtatg tcacaggccg 3420

gccggatatg cagaagcacc gcgtggtggc atggctgtcg cagcacaact tcccccacgg 3480

cgtcgtctcc ttctgcgacg gcctcaccca cgacccacta cgccagaagg caatgtttct 3540

gcagagcctg gtgcaggagg tagaactgaa catcgtggcc ggttatgggt ctcccaaaga 3600

tgtggctgta tacgcggcgc tggggctgtc cccgagccag acctacatcg tgggccgtgc 3660

cgtgcggaag ctacaggcgc agtgccagtt cctgtcagac ggctatgtgg cccacctggg 3720

ccagctggaa gcgggctcgc actcgcatgc ctcctcggga cccccgagag ctgccttggg 3780

caagagcagc tatggtgtgg ctgcccccgt ggacttcctg cgcaaacaga gccagctgct 3840

tcgctcgagg ggccccagcc aggcggagcg tgagggcccg ggaacaccac ccaccaccct 3900

ggcacggggc aaagcacgga gcatcagcct gaagctggac agcgaggagt gaggcccaca 3960

ccagcctgga cctgggttat ttattgacac acccaagggg cccgaggggc tgcgtgtggg 4020

aggctgggga cccagacttt tggccccagc gctggccccc ccagccccac accctatatc 4080

tccgtgtgct cctcggtgtt acttcccttt catatgaggg gacccagcgc cggggggagg 4140

gaggagggcg tgggcatggg cgcagaggct tttccagtgt gtataaatcc atgaaaataa 4200

acgccacctg cacccca 4217

<210> 3
<211> 4532
<212> DNA
<213> Homo sapiens

<400>  3
gcgcgacccg ggctggcggt ggtttccggt tccgcggggc tggcgggcga gcgggcggcc      60
gggatcggcg gcggcggcgg cggcggcggc gcttgacaga caatgagggc ggcggggcgg     120
cgttgagcgg cggcggcggc ggcggcggcg gcgagcgacg cggggcccgg gggcggggcg     180
gggcgccagc catggacaat cagtgcaccg tccaggtgag gttagagctg gggcatcgcg      240
cccaactgcg caagaagccc accacggagg ggttcactca cgactggatg gtgtttgtcc     300
gcggccccga gcaatgtgac atccagcact tcgtggagaa ggtggtcttc tggctgcacg     360
acagcttccc caagcccaga cgcgtgtgca aggagccccc ctacaaagta gaggagtcgg     420
ggtacgctgg cttcatcatg cccatcgagg tgcacttcaa aaacaaggag gagccgagga     480
aggtctgctt cacctacgac ctgttcctga acctggaagg caacccgccc gtgaaccacc     540
tgcgctgcga gaagctcacc ttcaacaacc ccaccacgga gttccggtac aagctcctgc     600
gggccggcgg ggtgatggta atgcccgaag gagcagacac ggtgtccagg cccagtcccg     660
actaccccat gttacccaca attccactct ctgccttctc tgaccccaag aagaccaaac     720
catcccacgg ctccaaggac gccaacaagg agagcagcaa gacctccaag ccacacaagg     780
tgaccaagga gcaccgggag cggccccgca aagactccga gagcaagagc tcctccaagg     840
agctggagcg tgagcaggcc aaaagctcca aggacacctc gcggaagctg ggcgagggcc     900
ggctgcccaa ggaggagaag gcgccaccgc ccaaggctgc cttcaaggaa cccaagatgg     960
ccctgaaaga gaccaagctg gaaagcacgt cccccaaggg tgggccccca cccccacccc    1020
cacccccacc ccgggcttcc agcaagcggc cggccaccgc cgactcgcca aagcccagcg    1080
ccaagaagca gaagaagagc agctcgaagg ggtcccggag tgctccaggc acctcgcccc    1140
gcacctcctc ctcctcctcc ttctcggaca agaagccggc caaggacaag agcagcacca    1200
gaggggagaa ggtgaaggcc gagagtgagc cccgggaggc caaaaaggcc ctggaggtgg    1260
aggagtccaa ctcagaggac gaggcctcct tcaagtccga gtctgcccag tcaagcccgt    1320
ccaactccag ctccagctca gactccagct cagactcaga cttcgagcca tcccagaacc    1380
acagccaagg acccctgcgc tccatggtgg aggacctgca gtccgaggag tccgacgagg    1440
acgactcttc gtcaggcgag gaggctgccg gcaagaccaa cccggggagg gactccaggt    1500
tgagcttcag cgacagcgag agtgacaaca gcgccgactc ctccctgccc agccgtgagc    1560
ccccaccccc ccagaagcca cccccgccca acagcaaggt gtcaggccgg aggagccccg    1620
agtcctgcag caagcctgag aagatcctca agaagggcac ctacgacaag gcctacacgg    1680
atgagctggt ggagctacac cggaggctga tggcgctgcg ggagcgcaac gtgctgcagc    1740
agattgtgaa tctgatcgag gagactggcc acttcaatgt caccaacacc accttcgact    1800
tcgacctctt ctccctggac gagaccaccg tgcgcaaact gcagagctgc ctggaggccg    1860

tggccacatg accctgggcc gcgtgccggg cccccaccgc cggggtcctg ggaggctgca 1920

gccaggcctc gcctgccctc ctctcccgtc tcgcctgccc gcagcactgc cttagcgaca 1980

gccctgtcct cgcccgcaca gcccgctgca ctttcctcgg cggctccctg cccactctcc 2040

ccgctccatc gccaggagcc ctggggccgg gctgggccac acgggagccg ccccccgggc 2100

tctctgggct cccctcagag agcggctggt gttgaaggca gctctgggca gagacagggc 2160

cgggggcccc tcgcccagcc ttgggaccag cccccactgc tcggcgggct gtgcggcgtc 2220

gctgccccag cctttctgag cagcggatcg acggctgggg ggcatctcca gcctcccgaa 2280

tggttcagaa cacaccctcc gcccaccctg gacctcagaa ggccctgcct gtgtgtctgc 2340

gtcgtcctcc gtgtagacct agcgccggct cctccccggc tcgcgctgcg ctggggtctg 2400

tgctgtatag ttctctctat tatatatgtg tgtgcactgt ctggtcaaga gcagtgtcca 2460

tgtgcgtttc tgtggcagca gtgcacgtgg ggtgggtggg gggtgcgtac ggtggggctt 2520

gtaggttaag atgtctctgc ggtggtgtgg ggctggaggc cgagccaggc acggcagctg 2580

gccctctcca ggcgcatgga ggaattctcc ctttgccgtt ccaaggatgc cgccgcctcc 2640

taccggggct cccggggggg tcagccaaca ctacaaaggg cagcgagtcc tcagggcgcc 2700

cgggagccac cagtctgctc tcgggctcta ctcagactag cgccaacagt ctccgcggac 2760

agactcgcat cagccgccct ggggctcggc ctcctcccat aggctcctct tcctctcttt 2820

cattcaagtc ttcaaacttt gaaaaaaata tgagcttttg ccaaataaga cgggatagtt 2880

tatggagttt tctgaggcgc attgaactct ggtcagcccc tcgagcgtgg gctcggccat 2940

cagcaggccc ggtgggttgg ccgggctggc cctcccaggc tgcctttctc tctggtcgcg 3000

gcctggcccg ccccggcctc caccgccgca attcatgctg gtgccgcgcc cgcagcccca 3060

ctcctggtat gcctgagatt ctccaggggc caagccggtc ctccttcccg gcccactctt 3120

ccagggccag gacgctcccg cggagccagc ccatcgtggc tgggggtccc aggggctgga 3180

ggcctgcctg gttagcctct gtttcccaga cattgactcg aggcgcctcc agtcctctca 3240

accccctgac tttcaagtcc tttcaagtcc ttgtgccctg accaagttat gaggaaaaga 3300

gggctgaggg cctggaggtg ggctcgcacc ccctcgaacc cacctagatg tctcctcctc 3360

cccagcctat gagttcagac ttggggggct ccatctgtcc ctccctgagg tccctcatct 3420

tgtccctgct ctgattctgt cagcagatcc tgaacactcc ttggtgggga cccatgcctc 3480

caaggctgcc cccaatgcca gctgtcctgg ggaaactccc cagaactctc ggggaaaggc 3540

ccgagaacct tcggaggaag cctccgggac tcctcgccac ctgctctgtt tctctcccgg 3600

ccccactgcc ttcaggtatc aggaagaggg caggcccctg tgccccctct ccccttccca 3660

gggcggccaa caatactcca ccatggggcg cttcctttct tctctggaaa ctaagacatt 3720

ggactgaagg aactggcctg aggggggctg accgggggcc cgtgctgctg ggagctctac 3780

gcacaagagg ccgccggggt cacggacagg aacctcagcc cagggtgctg gcctttctcc 3840

ttcgcaggag tcccccaaaa tccacacagc gactcgagca gccttccact cagccagagg 3900

agggtcgggc tcccccgcct gctctcggtg gtggcgtctg gcccttcctg ccgtttccaa 3960

ggcctccgga agcaccaacc cagttctcag ttacctcctc tggccggaag gcctcacgtt 4020

gtcctccaga gctgctctgg gcccagggct ggctccgtga ccacctgatg gacgtgctgg 4080

gcagcatggg ctcccaaggc agcatcccca gctgctctcc caccccaccc cagctgcccg 4140

gcggctgggc ccacgagggc tctggggaca cttccatagg aaaggggcct ggcactttgt 4200

gattgccacg tgtttcctgt taagccgcct gcccccagtg caaatctctg tgttttgctc 4260

tctcctgaac aaaaatgtaa accgacgccg gaaagcaaat ggtatcgaat acctttcttg 4320

cccataaggg tttttacaaa gaacgtgtct cactaggaac taggacactc atcggcccga 4380

gaccaactcc tggataaaag gaataaggag aatcgtgttt gtaacgagtt acaggattgt 4440

ttctttcctg gattttaaac ttttgttaaa ttgtgaaatt atggaggagt tttatagaaa 4500

aaaaaagtga aataaagtca gatgggaaag ca 4532

<210> 4
<211> 2993
<212> DNA
<213> Homo sapiens

<400> 4
actccactcc cggcacgccc ggtgccgcct tccggctcca gtccccgggc tcggcctcgg 60

cgaggtgtaa ttcgcagcgc gggccggccc cggaggctct cggcgagcgc ggcgcggtaa 120

caagtgggcg aggatgccgt acgagatcaa gaaggtgttc gccagcctcc cgcaggtgga 180

gaggggcgtc tccaagatca tcggcggcga ccctaagggc aacaattttc tgtacaccaa 240

tggaaagtgc gtcatcctaa ggaacatcga caacccagcc cttgctgaca tctacacaga 300

gcacgcccat caggtggtgg tggccaagta tgcgcccagc ggattctaca ttgcctccgg 360

agatgtgtct gggaagctga ggatctggga taccacgcag aaggagcacc tgttgaagta 420

tgagtaccag cctttcgctg ggaagatcaa agacattgct tggactgaag acagtaagag 480

gatcgccgtg gtcggggaag gaagggagaa gtttggagca gtcttcctct gggatagtgg 540

ctcttctgtg ggcgagatta caggacacaa caaagtcatc aacagcgtgg acatcaagca 600

gagccggcca taccggctgg ccacgggaag cgatgataac tgcgcggcat tctttgaggg 660

acccccattc aagttcaagt tcacaattgg cgaccacagc cgctttgtca actgtgtgcg 720

attctctcct gatgggaaca gatttgccac agccagtgct gacggccaga tatacatcta 780

tgacgggaag actggggaga aggtgtgcgc gctgggcgga agcaaggccc acgacggtgg 840

gatttacgca attagttgga gtcccgacag cacccatttg ctttctgctt ctggggacaa 900

aacttccaag atttgggacg tcagcgtgaa ctccgtggtc agcacatttc ccatgggctc 960

cacggttctg gaccagcagc tgggctgcct atggcagaag gaccacctgc tcagtgtctc 1020

cctgtccggg tacatcaact atctggacag aaacaacccc agcaagcccc tgcacgtcat 1080

caagggtcac agtaaatcga tccagtgtct gacggtgcat aaaaacggcg gcaagtccta 1140

catttactct gggagccacg acggacacat taattactgg gattcagaga cgggggagaa 1200

cgactccttc gctgggaaag gccacacgaa ccaggtgtcc aggatgaccg tggatgagtc 1260

ggggcagctc atcagctgca gcatggacga caccgtgcgg tacaccagcc tcatgctgcg 1320

ggactacagc ggacaaggag ttgtgaaact ggacgttcag ccaaagtgcg tagccgtcgg 1380

ccccggggga tacgccgtgg tcgtgtgcat tggacagatt gtcctgctga aggatcagag 1440

gaagtgcttc agcatcgaca accccggcta cgagcccgaa gttgtggcag tgcaccccgg 1500

cggggacacg gtggcaattg ggggtgtgga cggcaacgtc cgcctgtatt ccatcctggg 1560

caccacgctg aaggatgagg gcaagctcct agaggccaag ggccccgtga ccgacgtggc 1620

ctactcccac gacggcgcct tcctcgcggt gtgcgacgcc agcaaggtgg tcacagtgtt 1680

cagcgttgct gacggctact cggagaacaa tgttttttat ggacaccatg caaaaatcgt 1740

ctgcctggcc tggtccccag acaatgaaca ctttgcctcc ggtggcatgg acatgatggt 1800

gtatgtttgg accctgagtg acccggaaac cagagtcaag atccaagatg cacaccggct 1860

gcaccatgtc agcagcctgg cctggctgga cgagcacacg ctggtcacga cctcccatga 1920

tgcctctgtc aaggagtgga caatcaccta ctgaggagcc ccacccccgc ctctggatgg 1980

accgaatcag ggactagagt ttaactgcag cggaacatgt catttctcta tttctgtgac 2040

gcgcccccat gcccccaccc caccacaaga ggcaggaggg cccagtcatg accctcgtct 2100

ctgcagggtg tctgtacacg ttcttctgaa agctttagac agtaacagtt tgcacatgaa 2160

aaataaagcg agcacctaaa caatgtgtgg agcataacta aaacccacag cccaaccaaa 2220

ccttgagaat gcgaaacatt ccagaggcag tagcctccaa agcacacaga gcccctggcc 2280

ccgccgcggc tctcactatc tgtcagggga ggttgtacag gtgaatgagc cggggggctc 2340

atgttcctgc ctgcagaaca tttctgtact agtgagaaga gggaatatgc attgcagttc 2400

agcaaagccg gaattctgtg ttgaacagat gtctgtctcc ctagtgtgtg actcacacct 2460

tgtggctgcc ttcagagcgc cacctccaga tcagatgggg acacacaacc cctggatatg 2520

tttcattgtc agattttgtg cttgatttta agaatggaat tgtgggtatc tttccttttt 2580

ttttaatgta tcttaactgt tgcctgtcag tgtttacaaa ctagtgcgtt gacggcaccg 2640

tgtccaagtt tttagaaccc ttgttagcca gaccgaggtg tcctggtcac cgtttcacca 2700

tcatgctttg atgttcccct gtctttccct cttctgctct caagagcaaa ggttaattta 2760

aggacaaaga tgaagtcact gtaaactaat ctgtcattgt ttttaccttc cttttctttt 2820

tcagtgcaga aattaaaagt aagtataaag caccgtgatt gggagtgttt ttgcgtgtgt 2880

cggaatcact ggtaaatgtt ggctgagaac aatccctccc cttgcacttg tgaaaacact 2940

ttgagcgctt taagagatta gcctgagaaa taattaaata tcttttctct tca 2993

<210> 5
<211> 2626
<212> DNA
<213> Homo sapiens

<400> 5
gcactccgct cctttcctgc gtcaccgcta cacatgcgcc ttgcaaggaa gggcgagatt 60

tcgggggcag ggcggggggc gggcctaggc cggggtctca cagcgactgc gcggacgggt 120

tcctgagtgg aacatggcga cttgcgccga aatcctgcgg agcgagttcc ccgaaattga 180

cggacaagtc ttcgactacg tgaccggcgt cttgcacagc ggcagcgcgg acttcgagtc 240

tgtggatgac ctggtggaag ctgtagggga actattgcaa gaggtgtccg gggacagcaa 300

ggatgacgcg ggcatcaggg ccgtgtgcca gcgcatgtac aacactctgc gtctggctga 360

gccacaaagc cagggaaata gccaggtgct actggacgcc cctatccagt tgtcaaagat 420

aacggagaac tacgactgtg gaaccaaact tccaggactg ctaaagaggg aacagtcctc 480

gacagtgaat gcaaagaagt tagagaaggc cgaggctcga cttaaggcaa agcaggagaa 540

gcgctcagag aaggacacgc tcaagaccag caaccctcta gtcttagaag aggcatcagc 600

cagccaggca ggcagcagaa aggagagtcg gttggaatca tctggcaaga acaaatccta 660

tgatgtgcga attgagaact ttgatgtgtc ttttggcgat agagtactgc tggctggagc 720

ggatgtgaac ctggcatggg gccgccgtta cgggctggtg gggcggaatg ggttggggaa 780

gacaacgtta ctgaagatgc tggccacccg gagtctgcgg gttccagccc acatttccct 840

gctgcacgtt gagcaagagg ttgctggaga tgacactcct gccctgcaga gtgtgctgga 900

gagtgacagt gtgcgagagg atttgctacg gagggagcgg gagctcactg cccagattgc 960

tgctggcagg gcggagggct ctgaagctgc agagctggca gaaatctatg ccaaactgga 1020

ggagattgag gctgacaagg cacctgccag ggcatcagtc attctcgctg ggcttggctt 1080

tacccctaaa atgcagcagc agcccacccg ggagttctca ggtggctgga ggatgaggct 1140

ggccctggcc cgggccctct ttgctaggcc agatcttctg ctgttagatg aacctacaaa 1200

catgctggat gtcagggcca tcctgtggct ggagaattac ctgcagacgt ggccctccac 1260

catcctagtc gtctcccacg accgcaactt cttgaatgcc atcgccacag acatcatcca 1320

cctgcacagc cagcggctag atggttaccg gggagacttt gagaccttca tcaagagtaa 1380

gcaggagcgg ctgctcaacc agcagcgtga atatgaggcg cagcagcagt atcgccagca 1440

catccaggtt ttcattgacc ggtttcgcta caatgccaac agggcctctc aagtgcagag 1500

taaactcaag atgctggaga agctgcctga gctgaagcct gtggacaagg aatcagaggt 1560

cgtaatgaag ttccctgatg ggtttgagaa gttctcgccg ccaattctgc agctagatga 1620

ggtggatttc tactacgatc cgaagcacgt catcttcagt cgcctctctg tgtctgctga 1680

tctcgagtct cgcatctgtg tggttggaga gaatggggct gggaagtcta ccatgctgaa 1740

gctgcttttg ggggacctgg cacctgttcg gggcatcaga cacgctcaca ggaatctgaa 1800

gattggctat ttcagccagc accatgtgga gcagctggac ctaaacgtca gtgctgtgga 1860

actgctggca cgcaagtttc ctgggcggcc tgaggaggag taccgtcacc agctgggtcg 1920

gtatggcatc tccggagaac tggccatgcg tcctcttgcc agcctgtctg ggggccagaa 1980

gagccgagtg gcctttgctc agatgactat gccctgcccc aacttctaca ttctggatga 2040

acccacaaac cacctggaca tggagaccat tgaggctctg ggccgtgccc tcaacaattt 2100

caggggtggt gtgattctgg tgtcccacga tgagcgcttt atcaggctgg tgtgccggga 2160

gttgtgggta tgcgaaggag gcggcgtcac ccgtgtggaa ggaggatttg accagtaccg 2220

cgccctcctc caggaacagt tccgccgcga aggcttcctc tagggccacc aggctgagga 2280

ctcgcccagg acatggactg gtctctcaga cccctgggcc accatgtagg ccaccactcc 2340

aggccgtgga cttcccccaa cttggggaca gccttattcc caaatgtctc tatccttttg 2400

actggagcat cttctgcaca accttgggag cccatccaag ggttggtgag gactggtctc 2460

ccgggggtgg gggtctgggg ggtaccctct ggggttatag attcccccac tgccccagct 2520

ctgactggac cccaagtggc tgctatgtaa attaaatctc tccccgcgtc tcaaaaaaaa 2580

aaaaaaaaaa aaaaaaaaaa aaaaaaaaaa aaaaaaaaaa aaaaaa 2626

<210> 6
<211> 758
<212> DNA
<213> Homo sapiens

<400> 6
gtctgaaggc cgaggccaag atggcggtgc tgtcagctcc tggcctgcgc ggcttccgga 60

tccttggtct gcgctccagc gtgggcccgg ctgtgcaggc acgaggtgtc catcagagcg 120

tggccaccga tggcccaagc agcacccagc ctgccctgcc aaaggccaga gccgtggctc 180

ccaaacccag cagccggggc gagtatgtgg tggccaagct ggatgacctc gtcaactggg 240

cccgccggag ttctctgtgg cccatgacct tcggcctggc ctgctgcgcc gtggagatga 300

tgcacatggc agcaccccgc tacgacatgg accgctttgg cgtggtcttc cgcgccagcc 360

cgcgccagtc cgacgtcatg atcgtggccg gcacactcac caacaagatg gccccagcgc 420

ttcgcaaggt ctacgaccag atgccggagc cgcgctacgt ggtctccatg gggagctgcg 480

ccaacggagg aggctactac cactattcct actcggtggt gaggggctgc gaccgcatcg 540

tgcccgtgga catctacatc ccaggctgcc cacctacggc cgaggccctg ctctacggca 600

tcctgcagct gcagaggaag atcaagcggg agcggaggct gcagatctgg taccgcaggt 660

agcgccgccg ccgccgccgc cggagcctgt cgccgtcctg tccccagcct gcttgtgtcc 720

cgtgaggttg tcaataaacc tgccctcggg ctgccgcc 758

<210> 7
<211> 2909
<212> DNA
<213> Homo sapiens

<400> 7
gccagcggcg cggtcgggtg agaggccgcg gcggcaggtc cacctgggct tgcgaaggca 60

cagattcccc gtccacagct cacgaccaga tgcaccagca ggagtccaca tcgaggacgt 120

cctccgggca ctcccacgac cagtgaccag gagttaaact ttgggatgtg cccgtgatgt 180

tggaccacaa ggacttagag gccgaaatcc accccttgaa aaatgaagaa agaaaatcgc 240

aggaaaatct gggaaatcca tcaaaaaatg aggataacgt gaaaagcgcg cctccacagt 300

cccggctctc ccggtgccga gcggcggcgt tttttctttc attgtttctc tgcctttttg 360

tggtgttcgt cgtctcattc gtcatcccgt gtccagaccg gccggcgtca cagcgaatgt 420

ggaggataga ctacagtgcc gctgttatct atgactttct ggctgtggat gatataaacg 480

gggacaggat ccaagatgtt ctttttcttt ataaaaacac caacagcagc aacaatttca 540

gccgatcctg tgtggacgaa ggcttttcct ctccctgcac ctttgcagct gctgtgtcgg 600

gggccaacgg cagcacgctc tgggagagac ctgtggccca agacgtggcc ctcgtggagt 660

gtgctgtgcc ccagccaaga ggcagtgagg caccttctgc ctgcatcctg gtgggcagac 720

ccagttcttt cattgcagtc aacttgttca caggggaaac cctgtggaac cacagcagca 780

gcttcagcgg gaatgcgtcc atcctgagcc ctctgctgca ggtgcctgat gtggacggcg 840

atggggcccc agacctgctg gttctcaccc aggagcggga ggaggttagt ggccacctct 900

actccggcag caccgggcac cagattggcc tcagaggcag ccttggtgtg gacggggaaa 960

gtggcttcct ccttcacgtc accaggacag gtgcccacta catcctcttt ccctgcgcaa 1020

gctccctctg cggctgctct gtgaagggtc tctacgagaa ggtgaccggg agcggcggcc 1080

cgttcaagag tgacccgcac tgggagagca tgctcaatgc caccacccgc aggatgcttt 1140

cccacagctc tggagcagtg cgctacctga tgcatgtccc agggaacgcc ggtgcagatg 1200

tgcttcttgt gggctcagag gccttcgtgc tgctggacgg gcaggagctg acgcctcgct 1260

ggacacccaa ggcagcccat gtcctgagaa aacccatctt cggccgctac aaaccagaca 1320

ccttggctgt agccgttgaa aacggaactg gcaccgacag acagatcctg tttctggacc 1380

ttggcactgg agccgtcctg tgtagcctag ccctcccgag cctccctggg ggtccactgt 1440

ccgccagcct gccgaccgca gaccaccgct cagccttctt cttctggggc ctccacgagc 1500

tggggagcac cagcgagacg gagaccgggg aggcccggca cagcctgtac atgttccacc 1560

ccaccctgcc gcgcgtgctg ctggagctgg ccaatgtctc tacccacatt gtcgcctttg 1620

acgccgtcct gtttgagcca agccgccacg ccgcctacat ccttctgaca ggcccggcag 1680

actcagaggc acccggcctg gtctctgtga tcaagcacaa ggtgcgggac cttgtcccaa 1740

gcagcagggt ggtccgcctg ggtgagggtg ggccagacag tgaccaagcc atcagggacc 1800

ggttctcccg gctgcggtac cagagtgagg cgtagaggca cgccagccag agcctgtgga 1860

gagactccgc ctgctgacac taaacgtcct gggaagtggg cccttccctg ggtctctgca 1920

ctgactcccc cactcctgac cctggtgatg gtcgccactg ggcagcagca gccttaccag 1980

tcctccatga tcacacccag ggacctgcat gggtgagggg acaccctggg cctctctccc 2040

gcccagcatc ctccctgagt ccccacacag ggcctcactc tgcaccccac cagggtcccg 2100

ctcacaccag gcagccttca tagtggtctc cctggccacc ttgggcagag ctgggtcatg 2160

cagcacccca tccttacccg gtgccctctc cttgccagct tctccccagg ccagagcggc 2220

catcgcgtag aaagaaccag ggtgtccccg ggacaggccg tcccccaccc catcctgtag 2280

aagtccattc cccttttccc tcctgtgctc tgtcccccaa ggagtcatgg aactcagggt 2340

actgggcctc aacgggaacc tgagacagct ccagcttcgc agcccttccc ggagctacag 2400

ggggatcctc tagcatgggg ggtgtgactt ggttcctttg accaggtcct gtgaggaagc 2460

ctggagcaag ggtctccccc agcaggatgg gtggggcctg ctctggagct gagcccgtgg 2520

ccgctcacag gtgtccttag tggtgttgca gctgtctact ggctgcatgt gctgtgaata 2580

tcccaaggaa ctggctgtgg aatgcgtgtt tgggtcagtc tgtgccctct cagtagacac 2640

tggagctgct ctgtccctga agaggccccg tgccccaggc atggcaagcg cctgcctctc 2700

cccttccggt gctcacacgc ccacgccgtg ccacccgatg caggactcac ctctgtgcct 2760

tgctgctcct gaggcccaag ggcagccatg gtgctctgta ctgctcgggc cgcccaggtc 2820

acagagcctg agcttcgtag ccaaagcagc ctgatgaccc acccaccaag gaagaaagca 2880

gaataaacat ttttgcactg cctgaaaaa 2909

<210> 8
<211> 3433
<212> DNA
<213> Homo sapiens

<400> 8
cggctcagag ctccggaccg cgggcggagg ggaggggcag ggggcggtgc cacggcctgc 60

cagcccgccc gcccgcccgc cagccagccc tccccgcggc cggctcggct ccttggcgct 120

gcctggggtc ctttccgccc ggtccccgct tgccagcccc cgctgctctg tgccctgtcc 180

ggccaggcct ggagccgaca ccaccgccat catgccggcc gtgtccaagg gcgatgggat 240

gcgggggctc gcggtgttca tctccgacat ccggaactgt aagagcaaag aggcggaaat 300

taagagaatc aacaaggaac tggccaacat ccgctccaag ttcaaaggag acaaagcctt 360

ggatggctac agtaagaaaa aatatgtgtg taaactgctt ttcatcttcc tgcttggcca 420

tgacattgac tttgggcaca tggaggctgt gaatctgttg agttccaata aatacacaga 480

gaagcaaata ggttacctgt tcatttctgt gctggtgaac tcgaactcgg agctgatccg 540

cctcatcaac aacgccatca agaatgacct ggccagccgc aaccccacct tcatgtgcct 600

ggccctgcac tgcatcgcca acgtgggcag ccgggagatg ggcgaggcct ttgccgctga 660

catcccccgc atcctggtgg ccggggacag catggacagt gtcaagcaga gtgcggccct 720

gtgcctcctt cgactgtaca aggcctcgcc tgacctggtg cccatgggcg agtggacggc 780

gcgtgtggta cacctgctca atgaccagca catgggtgtg gtcacggccg ccgtcagcct 840

catcacctgt ctctgcaaga agaacccaga tgacttcaag acgtgcgtct ctctggctgt 900

gtcgcgcctg agccggatcg tctcctctgc ctccaccgac ctccaggact acacctacta 960

cttcgtccca gcaccctggc tctcggtgaa gctcctgcgg ctgctgcagt gctacccgcc 1020

tccagaggat gcggctgtga aggggcggct ggtggaatgt ctggagactg tgctcaacaa 1080

ggcccaggag ccccccaaat ccaagaaggt gcagcattcc aacgccaaga acgccatcct 1140

cttcgagacc atcagcctca tcatccacta tgacagtgag cccaacctcc tggttcgggc 1200

ctgcaaccag ctgggccagt tcctgcagca ccgggagacc aacctgcgct acctggccct 1260

ggagagcatg tgcacgctgg ccagctccga gttctcccat gaagccgtca agacgcacat 1320

tgacaccgtc atcaatgccc tcaagacgga gcgggacgtc agcgtgcggc agcgggcggc 1380

tgacctcctc tacgccatgt gtgaccggag caatgccaag cagatcgtgt cggagatgct 1440

gcggtacctg gagacggcag actacgccat ccgcgaggag atcgtcctga aggtggccat 1500

cctggccgag aagtacgccg tggactacag ctggtacgtg gacaccatcc tcaacctcat 1560

ccgcattgcg ggcgactacg tgagtgagga ggtgtggtac cgtgtgctac agatcgtcac 1620

caaccgtgat gacgtccagg gctatgccgc caagaccgtc tttgaggcgc tccaggcccc 1680

tgcctgtcac gagaacatgg tgaaggttgg cggctacatc cttggggagt ttgggaacct 1740

gattgctggg gacccccgct ccagcccccc agtgcagttc tccctgctcc actccaagtt 1800

ccatctgtgc agcgtggcca cgcgggcgct gctgctgtcc acctacatca agttcatcaa 1860

cctcttcccc gagaccaagg ccaccatcca gggcgtcctg cgggccggct cccagctgcg 1920

caatgctgac gtggagctgc agcagcgagc cgtggagtac ctcaccctca gctcagtggc 1980

cagcaccgac gtcctggcca cggtgctgga ggagatgccg cccttccccg agcgcgagtc 2040

gtccatcctg gccaagctga aacgcaagaa ggggccaggg gccggcagcg ccctggacga 2100

tggccggagg gaccccagca gcaacgacat caacgggggc atggagccca cccccagcac 2160

tgtgtcgacg ccctcgccct ccgccgacct cctggggctg cgggcagccc ctcccccggc 2220

agcacccccg gcttctgcag gagcagggaa ccttctggtg gacgtcttcg atggcccggc 2280

cgcccagccc agcctggggc ccacccccga ggaggccttc ctcagcccag gtcctgagga 2340

catcggccct cccattccgg aagccgatga gttgctgaat aagtttgtgt gtaagaacaa 2400

cggggtcctg ttcgagaacc agctgctgca gatcggagtc aagtcagagt tccgacagaa 2460

cctgggccgc atgtatctct tctatggcaa caagacctcg gtgcagttcc agaatttctc 2520

acccactgtg gttcacccgg gagacctcca gactcagctg gctgtgcaga ccaagcgcgt 2580

ggcggcgcag gtggacggcg gcgcgcaggt gcagcaggtg ctcaatatcg agtgcctgcg 2640

ggacttcctg acgcccccgc tgctgtccgt gcgcttccgg tacggtggcg ccccccaggc 2700

cctcaccctg aagctcccag tgaccatcaa caagttcttc cagcccaccg agatggcggc 2760

ccaggatttc ttccagcgct ggaagcagct gagcctccct caacaggagg cgcagaaaat 2820

cttcaaagcc aaccacccca tggacgcaga agttactaag gccaagcttc tggggtttgg 2880

ctctgctctc ctggacaatg tggaccccaa ccctgagaac ttcgtggggg cggggatcat 2940

ccagactaaa gccctgcagg tgggctgtct gcttcggctg gagcccaatg cccaggccca 3000

gatgtaccgg ctgaccctgc gcaccagcaa ggagcccgtc tcccgtcacc tgtgtgagct 3060

gctggcacag cagttctgag ccctggactc tgccccgggg gatgtggccg gcactgggca 3120

gccccttgga ctgaggcagt tttggtggat gggggacctc cactggtgac agagaagaca 3180

ccagggtttg ggggatgcct gggactttcc tccggccttt tgtattttta tttttgttca 3240

tctgctgctg tttacattct ggggggttag ggggagtccc cctccctccc tttccccccc 3300

aagcacagag gggagagggg ccagggaagt ggatgtctcc tcccctccca ccccaccctg 3360

ttgtagcccc tcctaccccc tccccatcca ggggctgtgt attattgtga gcgaataaac 3420

agagagacgc taa 3433

<210> 9
<211> 80
<212> DNA
<213> Artificial sequence

<220>
<223> chemically synthesized

<400> 9
accactggct gcctcaccta ctccccacac cagatcggca tcaagcagat cctgccacac 60

cagatgacca cggcagggcc 80

<210>  10
<211>  80
<212>  DNA
<213>  Artificial sequence

<220>
<223>  chemically synthesized

<400>  10
cactccagcc tctttctgga ggagctggag atgctggtgc cctcaacacc cacctctact        60

agcggtgcct tctggaaggg                                                    80


<210>  11
<211>  80
<212>  DNA
<213>  Artificial sequence

<220>
<223>  chemically synthesized

<400>  11
atctgatcga ggagactggc cacttcaatg tcaccaacac caccttcgac ttcgacctct        60

tctccctgga cgagaccacc                                                    80


<210>  12
<211>  80
<212>  DNA
<213>  Artificial sequence

<220>
<223>  chemically synthesized

<400>  12
agcctggcct ggctggacga gcacacgctg gtcacgacct cccatgatgc ctctgtcaag        60

gagtggacaa tcacctactg                                                    80


<210>  13
<211>  80
<212>  DNA
<213>  Artificial sequence

<220>
<223>  chemically synthesized

<400>  13
tgactatgcc ctgccccaac ttctacattc tggatgaacc cacaaaccac ctggacatgg        60

agaccattga ggctctgggc                                                    80


<210>  14
<211>  80
<212>  DNA
<213>  Artificial sequence

<220>
<223>  chemically synthesized

<400>  14
actattccta ctcggtggtg aggggctgcg accgcatcgt gcccgtggac atctacatcc      60

caggctgccc acctacggcc                                                  80


<210>  15
<211>  80
<212>  DNA
<213>  Artificial sequence

<220>
<223>  chemically synthesized

<400>  15
tggcaccgac agacagatcc tgtttctgga ccttggcact ggagccgtcc tgtgtagcct      60

agccctcccg agcctccctg                                                  80


<210>  16
<211>  80
<212>  DNA
<213>  Artificial sequence

<220>
<223>  chemically synthesized

<400>  16
agcattccaa cgccaagaac gccatcctct tcgagaccat cagcctcatc atccactatg      60

acagtgagcc caacctcctg                                                  80


<210>  17
<211>  80
<212>  DNA
<213>  Artificial sequence

<220>
<223>  chemically synthesized

<400>  17
ctttgagtct atcaaaagga aacttccttt cctcaactgg gatgcctttc ctaagctgaa      60

aggactgagg agcgcaactc                                                  80


<210>  18
<211>  80
<212>  DNA
<213>  Artificial sequence

<220>
<223>  chemically synthesized

<400>  18
tggaagagag ctatgacctc aaggacacgt tgagaaccat gggaatggtg gatatcttca      60

atggggatgc agacctctca                                                  80

<210> 19
<211> 80
<212> DNA
<213> Artificial sequence

<220>
<223> chemically synthesized

<400> 19
cctcagcagt gccagcagaa atatcctcct gtgacacctt ccccaccctg ccagtcaaag 60

tatccaccca agagcaagta 80


<210> 20
<211> 80
<212> DNA
<213> Artificial sequence

<220>
<223> chemically synthesized

<400> 20
gttttcagct gctcagaatt catctgaaga gagacttaag atgaaagcaa atgattcagc 60

tcccttatac ccccattaaa 80


<210> 21
<211> 80
<212> DNA
<213> Artificial sequence

<220>
<223> chemically synthesized

<400> 21
ccgtgtctca agatacgtcc tgtggatccg tgaaacaatc cgaaaatatg aaacccagca 60

gcaaaaatgg ttgaagggcc 80


<210> 22
<211> 80
<212> DNA
<213> Artificial sequence

<220>
<223> chemically synthesized

<400> 22
tccgaagaag agtggaccaa ctgaagagtg atcaatctcg gttggattcg gaactgaaga 60

acatgcagga catggtggag 80


<210> 23
<211> 80
<212> DNA
<213> Artificial sequence

<220>
<223>  chemically synthesized

<400>  23
gcagcccttc gaggtgctca tcatcgcgtc agacgacggc ttcaaggccg tggttgggga      60

cgcccagtac caccacttcc                                                  80


<210>  24
<211>  80
<212>  DNA
<213>  Artificial sequence

<220>
<223>  chemically synthesized

<400>  24
gttcctgtta aaggtcaaga cactgtcaaa ggccgtgttc cattcaatgg acaagatccc      60

gttaaaggac aagtttcagt                                                  80


<210>  25
<211>  80
<212>  DNA
<213>  Artificial sequence

<220>
<223>  chemically synthesized

<400>  25
cccaagctgt tctacgccga ccaccccttc atcttcctag tgcgggacac ccaaagcggc      60

tccctgctat tcattgggcg                                                  80


<210>  26
<211>  80
<212>  DNA
<213>  Artificial sequence

<220>
<223>  chemically synthesized

<400>  26
gctccttcct ggtcaagaag catttcaacg cctccaaaaa gccaaactac agcgaactgg      60

acacacatac agtgattatt                                                  80


<210>  27
<211>  80
<212>  DNA
<213>  Artificial sequence

<220>
<223>  chemically synthesized

<400>  27
cacggctctt catggacctt cagtgcactc agctgcaaga ttgtggcctt tatggccgtg      60

ctcttttgct tccatgcggc                                                  80

<210>  28
<211>  80
<212>  DNA
<213>  Artificial sequence

<220>
<223>  chemically synthesized

<400>  28
cagacagttc taattgttgg aacgttgctc tatgcatgct attgggtcat gcttttgacg      60

ctgtatgtag ttctcatcaa                                                  80


<210>  29
<211>  80
<212>  DNA
<213>  Artificial sequence

<220>
<223>  chemically synthesized

<400>  29
ttttgtcgca atcatatact gtttctgcaa tggcgaggta caagctgaga tcaagaaatc      60

ttggagccgc tggacactgg                                                  80


<210>  30
<211>  80
<212>  DNA
<213>  Artificial sequence

<220>
<223>  chemically synthesized

<400>  30
tgatgtggaa gcttttcaac aggaactggc tctgcttaag atagaggatg cacctggaga      60

tggtcctgat gtcagggagg                                                  80


<210>  31
<211>  80
<212>  DNA
<213>  Artificial sequence

<220>
<223>  chemically synthesized

<400>  31
tgcatttgat ggcctgtatt ttctccgcac tgagaatggt gttatctacc agaccttctg      60

tgacatgacc tctgggggtg                                                  80


<210>  32
<211>  80
<212>  DNA
<213>  Artificial sequence

<220>
<223>  chemically synthesized

<400>  32
agatgccact ccccagaatc ccttcttgaa gtccagacct caggttaggc caaaaccagc      60

tccttccccc aaaacggagc                                                  80


<210>  33
<211>  80
<212>  DNA
<213>  Artificial sequence

<220>
<223>  chemically synthesized

<400>  33
acccagtggt ctgttgactc ccttacactt ggagggcctt gtccagttcc aagatgtctc      60

ctttgcctac ccaaaccgcc                                                  80


<210>  34
<211>  80
<212>  DNA
<213>  Artificial sequence

<220>
<223>  chemically synthesized

<400>  34
aagacatacc acgtaggaga acagtggcag aaggaatatc tcggtgccat ttgctcctgc      60

acatgctttg gaggccagcg                                                  80


<210>  35
<211>  80
<212>  DNA
<213>  Artificial sequence

<220>
<223>  chemically synthesized

<400>  35
gaaagctttg aggagtcctg gacacccaac tacaagcagt gttcatggag ttcattgaat      60

tatggcatag atcttgggaa                                                  80


<210>  36
<211>  80
<212>  DNA
<213>  Artificial sequence

<220>
<223>  chemically synthesized

<400>  36
gtgagttccc ggagtgagtt gaaccaggtg gaccaagtgg gctacgtgac ctatgacatc      60

ctgcagtgcc ctgaggacta                                                  80

<210>  37
<211>  80
<212>  DNA
<213>  Artificial sequence

<220>
<223>  chemically synthesized

<400>  37
ctatatgaag gatgtgaagg tcagcctctt caagaaggac aaggctatgc tcttcaccta        60

tgatcagtac caggaaaata                                                    80


<210>  38
<211>  80
<212>  DNA
<213>  Artificial sequence

<220>
<223>  chemically synthesized

<400>  38
gctgtcattg gtgctgtcct ggctgtgttt ggaggtattc taatgccagt tggagacctg        60

cttatccaga agacaattaa                                                    80


<210>  39
<211>  80
<212>  DNA
<213>  Artificial sequence

<220>
<223>  chemically synthesized

<400>  39
ggaaacggtc caggagctga tccccctggc caaggagatg atggcccaga agcgcaaggg        60

gaagatggtg aagctgtacg                                                    80


<210>  40
<211>  80
<212>  DNA
<213>  Artificial sequence

<220>
<223>  chemically synthesized

<400>  40
gaagacattg gaggcaaaaa gctgactgag atcatcaaca cgcagcatga gaatgtcaaa        60

tacctgccag ggcacaagtt                                                    80


<210>  41
<211>  80
<212>  DNA
<213>  Artificial sequence

<220>
<223>  chemically synthesized

<400>  41
acactggcag tctaccaaca gatcctcacc agtatgcctt ccagaaacgt gatccaaata      60

tccaacgacc tggagaacct                                                  80


<210>  42
<211>  80
<212>  DNA
<213>  Artificial sequence

<220>
<223>  chemically synthesized

<400>  42
ccactgggca cggattccct caagaacctg accctgaggg acttgagcct gaggggaaac      60

tccgagacgt cgtcggacac                                                  80


<210>  43
<211>  80
<212>  DNA
<213>  Artificial sequence

<220>
<223>  chemically synthesized

<400>  43
ctgggtggtg tggcacatac cctgcagaag accctccaga ccaccatctc ggctgtgaca      60

tgggcacctg cagctgtgct                                                  80


<210>  44
<211>  80
<212>  DNA
<213>  Artificial sequence

<220>
<223>  chemically synthesized

<400>  44
cctgcctagc agtgagcaga tgccaaatga ccaggaagag gagtcctttg cagagggtca      60

ttccgaagcg tccctcgcca                                                  80


<210>  45
<211>  80
<212>  DNA
<213>  Artificial sequence

<220>
<223>  chemically synthesized

<400>  45
atttagtgtc tccagactct cctagatcca tagacagcaa cagtgagatt gtctcatttg      60

gaagtccatg ctccattaat                                                  80

<210>  46
<211>  80
<212>  DNA
<213>  Artificial sequence

<220>
<223>  chemically synthesized

<400>  46
ggataagaag cgattcagct ttgtcttcaa gcctgtcttc aacacctcac tgctctttct        60

acagtgtgag ctgacgctgt                                                    80


<210>  47
<211>  80
<212>  DNA
<213>  Artificial sequence

<220>
<223>  chemically synthesized

<400>  47
aagggtggtc caactgccaa agtcaagcaa ggccacatca gcttcaaggt tatttccagc        60

gatgatctca ctgagctcta                                                    80


<210>  48
<211>  80
<212>  DNA
<213>  Artificial sequence

<220>
<223>  chemically synthesized

<400>  48
caagaaggca aaaaggtcat ccaaggagga agccgagatg gcctacaaag acttcctgct        60

ccagtccagc accgtggccg                                                    80


<210>  49
<211>  80
<212>  DNA
<213>  Artificial sequence

<220>
<223>  chemically synthesized

<400>  49
ggtagcagca gcaatttcca catcaatgta gaagagtcag tggatggaca ggtggtttct        60

tcccacaaga gagaaatcta                                                    80


<210>  50
<211>  80
<212>  DNA
<213>  Artificial sequence

<220>
<223>  chemically synthesized

<400>  50
tgcagactca gtggagaagg agttggacca gtcaacatct ctgttgtcac aagcagtgtt      60

tcctctggat atggcagtgg                                                  80


<210>  51
<211>  80
<212>  DNA
<213>  Artificial sequence

<220>
<223>  chemically synthesized

<400>  51
gagatcaaag actacagtcc ctacttcaag accattgagg acctgaggaa caagattctc      60

acagccacag tggacaatgc                                                  80


<210>  52
<211>  80
<212>  DNA
<213>  Artificial sequence

<220>
<223>  chemically synthesized

<400>  52
caaagccttg gtcaaaaagt atcaggaagg cctcatcaca cttacagaac ttgctgattc      60

tttgctgagc cggttagtcc                                                  80


<210>  53
<211>  80
<212>  DNA
<213>  Artificial sequence

<220>
<223>  chemically synthesized

<400>  53
cccacctgga ttctatggag tgaactgtga caaagcaaac tgctcaacca cctgctttaa      60

tggagggacc tgtttctacc                                                  80


<210>  54
<211>  80
<212>  DNA
<213>  Artificial sequence

<220>
<223>  chemically synthesized

<400>  54
aaggagcagg ccatgtttga taagaaggtg cagctccaga gaatggtaga ccaaaggtcg      60

gtgatttcag atgaaaagaa                                                  80

<210>  55
<211>  80
<212>  DNA
<213>  Artificial sequence

<220>
<223>  chemically synthesized

<400>  55
tagattctag tgcattcaag cacaatggca cggttgaatg taaggcttac aacgatgtgg      60

gcaagacttc tgcctatttt                                                  80


<210>  56
<211>  80
<212>  DNA
<213>  Artificial sequence

<220>
<223>  chemically synthesized

<400>  56
ctacagtgac ttgacaatga aaaccagcga caagttcaag tttgtcttcc gagaaaagat      60

gggcaggatt gttgattatt                                                  80


<210>  57
<211>  80
<212>  DNA
<213>  Artificial sequence

<220>
<223>  chemically synthesized

<400>  57
atggcgacaa gaagattgtc cccaagaaga agaagcccct gaagttgggg cccatcaaga      60

agaaggacct gaagaagctt                                                  80


<210>  58
<211>  80
<212>  DNA
<213>  Artificial sequence

<220>
<223>  chemically synthesized

<400>  58
gaatttgtac gagacctgct tctatctcct gaagaaaact gtggcattct ggaatgggaa      60

gatagggaac aaggaatttt                                                  80


<210>  59
<211>  80
<212>  DNA
<213>  Artificial sequence

<220>
<223>  chemically synthesized

<400>  59
agcattgtcc gagaggacca gaccctgaaa cctggcaaag tcgatgtgaa gacagacaaa      60

tgggatttct actgccagtg                                                  80


<210>  60
<211>  80
<212>  DNA
<213>  Artificial sequence

<220>
<223>  chemically synthesized

<400>  60
ccattctttg ggtatgggac attcctctga tcctaatgca gtgatgtatc caacctatgg      60

aaatggagat ccccaaaatt                                                  80


<210>  61
<211>  80
<212>  DNA
<213>  Artificial sequence

<220>
<223>  chemically synthesized

<400>  61
tttgtgttcc cttacccata tccatttcgc ccacttccac caattccatt tccaagattt      60

ccatggttta gacgtaattt                                                  80


<210>  62
<211>  80
<212>  DNA
<213>  Artificial sequence

<220>
<223>  chemically synthesized

<400>  62
gaggaggaga ccgtggacgg acgcaagtgc aggagtttag ccacttggga gaatgagaac      60

aagatccact gcacccaaac                                                  80


<210>  63
<211>  80
<212>  DNA
<213>  Artificial sequence

<220>
<223>  chemically synthesized

<400>  63
cgcacaggga atggattgtt ggagagagta actaggattc tagcttctct ggattttgct      60

cagaacttca tcacaaacaa                                                  80

<210>  64
<211>  80
<212>  DNA
<213>  Artificial sequence

<220>
<223>  chemically synthesized

<400>  64
aagatcaagg ccataaccca ggagaccatc aacggaagat tagttctttg tcaagtgaat        60

gaaatccaaa agcacgcatg                                                    80


<210>  65
<211>  80
<212>  DNA
<213>  Artificial sequence

<220>
<223>  chemically synthesized

<400>  65
atgcccagaa ggatgtggat gctgtggaca aggtgatgaa ggagctagac gagaatggag        60

acggggaggt ggacttccag                                                    80


<210>  66
<211>  80
<212>  DNA
<213>  Artificial sequence

<220>
<223>  chemically synthesized

<400>  66
tgcgaaatgg aagcctgcac atcattgatg acttcgagtc taaattcacg ttccattctg        60

tggaagactt tccccctccg                                                    80


<210>  67
<211>  80
<212>  DNA
<213>  Artificial sequence

<220>
<223>  chemically synthesized

<400>  67
ctgctctttg tctacgcaga tgattgcctt gctcagtgtg gcaaagattg caaatcttac        60

tgctgtgatg gaaccacgcc                                                    80


<210>  68
<211>  80
<212>  DNA
<213>  Artificial sequence

<220>
<223>  chemically synthesized

<400>  68
tgtaccacgc catcttcctg gaagagctga ccaccttgga gctgattgag aagattgcca      60

acctgtacag catctccccc                                                  80


<210>  69
<211>  80
<212>  DNA
<213>  Artificial sequence

<220>
<223>  chemically synthesized

<400>  69
gttgggcata gcattgctct tttgcatcct gtttacgctg gtctgtgggg cttctgggac      60

gtctaaacaa ccaaaagtaa                                                  80


<210>  70
<211>  80
<212>  DNA
<213>  Artificial sequence

<220>
<223>  chemically synthesized

<400>  70
tggcagcagt aagatcttcc ctggcaactg ggacaaccac tcccacaaga agaacttgtt      60

tgagacgccc atcctggctc                                                  80


<210>  71
<211>  80
<212>  DNA
<213>  Artificial sequence

<220>
<223>  chemically synthesized

<400>  71
caacccaatg acccaggagt ctacactcaa gtgtgcaagt tcaccaagtg gataaatgac      60

accatgaaaa agcatcgcta                                                  80


<210>  72
<211>  80
<212>  DNA
<213>  Artificial sequence

<220>
<223>  chemically synthesized

<400>  72
cttctggggg tcacagagca tgttctcgcc aacaatgatg tttcctgtga ccacccctct      60

aacaccgtgc cctctgggag                                                  80

<210>  73
<211>  80
<212>  DNA
<213>  Artificial sequence

<220>
<223>  chemically synthesized

<400>  73
ccatcccata gaagtccagc agacaggatt tgttaagtgc cagactttgt caggaagtca        60

aggagcttct gctttgtccg                                                    80


<210>  74
<211>  80
<212>  DNA
<213>  Artificial sequence

<220>
<223>  chemically synthesized

<400>  74
gactacgatg acacagacga tgacgaccct ttcaaccctc aggaatccaa gcgctttgtg        60

cagtggcagt cgtctatctg                                                    80


<210>  75
<211>  80
<212>  DNA
<213>  Artificial sequence

<220>
<223>  chemically synthesized

<400>  75
ttcagcgaat tgctctgctg cccaaaagac gttttctttg gaccaaagat ctctttcgtg        60

attccttgca acaatcaatg                                                    80


<210>  76
<211>  80
<212>  DNA
<213>  Artificial sequence

<220>
<223>  chemically synthesized

<400>  76
gggagacaag cacaagctga agaaatccga actcaaggag ctcatcaaca atgagctttc        60

ccatttctta gaggaaatca                                                    80


<210>  77
<211>  80
<212>  DNA
<213>  Artificial sequence

<220>
<223>  chemically synthesized

<400>  77
ctcattcagg tgatgtgctc tatgaactcc ttcagcatat tctgaagcag aggaaacctc      60

ggattctttt ttcaccattc                                                  80


<210>  78
<211>  80
<212>  DNA
<213>  Artificial sequence

<220>
<223>  chemically synthesized

<400>  78
aagaaataca gagaaggaca gtaatgctga gaacatgaat tctcaacctg agaaaacagt      60

tactactccc acagaaaaaa                                                  80


<210>  79
<211>  80
<212>  DNA
<213>  Artificial sequence

<220>
<223>  chemically synthesized

<400>  79
acaataagaa cgggttcaaa atgttcgact cctccaacta tcatggccaa gacctgcttt      60

tcaaggatgc caccgtccgg                                                  80


<210>  80
<211>  80
<212>  DNA
<213>  Artificial sequence

<220>
<223>  chemically synthesized

<400>  80
agtcctccat cactttgatt cttctagtca agagtcagtg ccaaaaagga gaaagtttag      60

tgaaccaaag gaacatatat                                                  80


<210>  81
<211>  80
<212>  DNA
<213>  Artificial sequence

<220>
<223>  chemically synthesized

<400>  81
atgtggtcca gcaaggaact gcagggtgtc ctcagacagc aaggtttcga taggaacatg      60

ctgggaacct tagctggggc                                                  80

<210>  82
<211>  80
<212>  DNA
<213>  Artificial sequence

<220>
<223>  chemically synthesized

<400>  82
gaacaactct ccagtgaacg ggaatagtag ctgtcaaatg gccttccctt ccagccagtc        60

tctgtaccgc acgtccggag                                                    80


<210>  83
<211>  80
<212>  DNA
<213>  Artificial sequence

<220>
<223>  chemically synthesized

<400>  83
tcatgtgctc tcatggaaca acagcattat gtcgaccaaa cttcagaatg tttctccagc        60

ccaagctact tgagaatatt                                                    80


<210>  84
<211>  80
<212>  DNA
<213>  Artificial sequence

<220>
<223>  chemically synthesized

<400>  84
agtaccagcc ttccaaatga aactctttca gagttagaga cacctgggaa atactcactt        60

acaccaccaa accactgggg                                                    80


<210>  85
<211>  80
<212>  DNA
<213>  Artificial sequence

<220>
<223>  chemically synthesized

<400>  85
gcttgaagac catggtgaga aaaaccagaa gcttgaagac catggtgtga aaatccttga        60

acccacccaa atacctgctc                                                    80


<210>  86
<211>  80
<212>  DNA
<213>  Artificial sequence

<220>
<223>  chemically synthesized

<400>  86
tctctattcc cagtataagg gacgaaactg cgaactcctt aaagctctat ctagccaaac      60

cgcttacgac cttgtatata                                                  80


<210>  87
<211>  80
<212>  DNA
<213>  Artificial sequence

<220>
<223>  chemically synthesized

<400>  87
ttccgcttgg agcccaagga tgggaaaggt gtgtttgcag tggatgggga attgatggtt      60

agcgaggccg tgcagggcca                                                  80


<210>  88
<211>  80
<212>  DNA
<213>  Artificial sequence

<220>
<223>  chemically synthesized

<400>  88
gcagacaagt tcctgcagct gtttggaacc aaaggtgtca agagggtgct gtgtcctatc      60

aactacccct tgtcgcccac                                                  80


<210>  89
<211>  80
<212>  DNA
<213>  Artificial sequence

<220>
<223>  chemically synthesized

<400>  89
cttggctgct cctctgacac catcgaggtc tcctgcaact tcactcatgg tggacagacg      60

tgtctcaagc ccatcacggc                                                  80


<210>  90
<211>  80
<212>  DNA
<213>  Artificial sequence

<220>
<223>  chemically synthesized

<400>  90
cccacgcatg agatccgctg caactctgag gtcaagtacg cctcggagaa gcatttccag      60

gacaaggtct tctatgcgcc                                                  80

<210>  91
<211>  80
<212>  DNA
<213>  Artificial sequence

<220>
<223>  chemically synthesized

<400>  91
gtccttcgat gaggtcaagc gccttcagaa gcagatcctg gaggtcaagt cctgcctgaa      60

gaacaagacc aaggaggcgg                                                  80


<210>  92
<211>  80
<212>  DNA
<213>  Artificial sequence

<220>
<223>  chemically synthesized

<400>  92
aaaccagcaa ccatcaaggt ctatgactac tacctaccag atgaacaggc aacaattcag      60

tattctgatc cctgtgaatg                                                  80


<210>  93
<211>  80
<212>  DNA
<213>  Artificial sequence

<220>
<223>  chemically synthesized

<400>  93
ggacaatttg cgatgacgag tggcaaaatt ctgatgccat tgtcttctgc cgcatgctgg      60

gttactccaa aggaagggcc                                                  80


<210>  94
<211>  80
<212>  DNA
<213>  Artificial sequence

<220>
<223>  chemically synthesized

<400>  94
tcaacgaaga attgtggccc aatcccttgc agttctacct tttgagtgaa ggggctcgtg      60

tagagaaagg aaaggaaaaa                                                  80


<210>  95
<211>  80
<212>  DNA
<213>  Artificial sequence

<220>
<223>  chemically synthesized

<400>  95
gatgctttct tctgtattgg ctgctctcat tggaattgca ggatctggct actgtgtcat        60

tgtggcagcc cttggcttag                                                    80


<210>  96
<211>  80
<212>  DNA
<213>  Artificial sequence

<220>
<223>  chemically synthesized

<400>  96
acaataacaa gaaaattgaa agatgggaaa ttagtggtgg agtgtgtcat gaacaatgtc        60

acctgtactc ggatctatga                                                    80


<210>  97
<211>  80
<212>  DNA
<213>  Artificial sequence

<220>
<223>  chemically synthesized

<400>  97
cttcagctgt ctgtacccag atggcgtctt ctatgacctg gacagctgca agcattccag        60

ctaccctgat tcagaggggg                                                    80


<210>  98
<211>  80
<212>  DNA
<213>  Artificial sequence

<220>
<223>  chemically synthesized

<400>  98
tgccagaaca ctattcaacc aagtgatgga aaaggagttt gaagacggca tcattaactg        60

gggaagaatt gtaaccatat                                                    80


<210>  99
<211>  80
<212>  DNA
<213>  Artificial sequence

<220>
<223>  chemically synthesized

<400>  99
ctcccggaac tggcaggact acggagttcg agaagtggac caagtgaaac gtctcacagg        60

cccaggactt agcgaggggc                                                    80

<210>  100
<211>  80
<212>  DNA
<213>  Artificial sequence

<220>
<223>  chemically synthesized

<400>  100
aaggacgtca acatcttcag ctttctttcc aagaaggaag agacctacaa cgacctcatc        60

aagcagatcg ggaagaaggc                                                    80


<210>  101
<211>  80
<212>  DNA
<213>  Artificial sequence

<220>
<223>  chemically synthesized

<400>  101
acatgataga cctacagctc tcatcaatgg ccatttgaat tcacttcaga gcaatccact        60

actgccagac cagaaggtct                                                    80


<210>  102
<211>  80
<212>  DNA
<213>  Artificial sequence

<220>
<223>  chemically synthesized

<400>  102
tctaagtttc ttttcctttt ctgtctgtat ctgtttttct ctgactgcct atatcttact        60

ttgtataccc atacataaat                                                    80


<210>  103
<211>  80
<212>  DNA
<213>  Artificial sequence

<220>
<223>  chemically synthesized

<400>  103
gtcatttatc ctgcagtgct ttgctgcaag ataacatcgc tgatgctgta gcttgtgcaa        60

agagggttgt ccgtgatcca                                                    80


<210>  104
<211>  80
<212>  DNA
<213>  Artificial sequence

<220>
<223>  chemically synthesized

<400>  104
catggtggag ctgctgctgc tgcagaacgc acaggtgcac cagttggtcc tgcagaactg      60

gatgctcaag gccctgcccc                                                  80


<210>  105
<211>  80
<212>  DNA
<213>  Artificial sequence

<220>
<223>  chemically synthesized

<400>  105
cttctctatg tgaagcagca gccttggtat tgtgaggtct accaatacag tgagtgtttt      60

ctggccaacc aaagcccata                                                  80


<210>  106
<211>  80
<212>  DNA
<213>  Artificial sequence

<220>
<223>  chemically synthesized

<400>  106
accgtggtgc cagtgcttgc tggtgtaggc ccactggatc cccagagcct caggcccaac      60

tcggaggagg tgagctgggg                                                  80


<210>  107
<211>  80
<212>  DNA
<213>  Artificial sequence

<220>
<223>  chemically synthesized

<400>  107
gactgactac aaacaatgca tggccgtgaa cttctttgga actgtggagg tcacaaagac      60

gtttttgcct cttcttagaa                                                  80


<210>  108
<211>  80
<212>  DNA
<213>  Artificial sequence

<220>
<223>  chemically synthesized

<400>  108
aagtcctatg aaactgctga taccagcagc caaggttcca ctataagtgt tgactcacaa      60

gatgaccaga tggagtctta                                                  80

<210>  109
<211>  80
<212>  DNA
<213>  Artificial sequence

<220>
<223>  chemically synthesized

<400>  109
aacactgatg ttcccatccc aacacactac tttgtggtgc tgaccagttg taaaaacaag      60

agccacacac cggaaaactg                                                  80


<210>  110
<211>  80
<212>  DNA
<213>  Artificial sequence

<220>
<223>  chemically synthesized

<400>  110
gccactgcaa gttccactgg tgctgctacg tcaagtgcaa gaagtgcacg gagatcgtgg      60

accagtttgt gtgcaagtag                                                  80


<210>  111
<211>  80
<212>  DNA
<213>  Artificial sequence

<220>
<223>  chemically synthesized

<400>  111
ggtgcctaca gccagctcaa agtggtctta gagaagctga gcaaggacac tcactgggta      60

cctgttagtt gggtcaggta                                                  80


<210>  112
<211>  80
<212>  DNA
<213>  Artificial sequence

<220>
<223>  chemically synthesized

<400>  112
aatcgtggat ggcagattac tgtaaagatg acaaggacat agagtcagct aaatcagaag      60

actggatggg ctcttcggtg                                                  80


<210>  113
<211>  80
<212>  DNA
<213>  Artificial sequence

<220>
<223>  chemically synthesized

<400>  113
acctacctct atgaggacag caatgtatgg cacgacatct cagacttcct cgtctataac        60

aagagccgtc cctccaatta                                                    80


<210>  114
<211>  80
<212>  DNA
<213>  Artificial sequence

<220>
<223>  chemically synthesized

<400>  114
ccggtcagcg ggatcctgtt caataatgaa atggacgact tcagctctcc cagcatcacc        60

aacgagtttg gggtaccccc                                                    80


<210>  115
<211>  80
<212>  DNA
<213>  Artificial sequence

<220>
<223>  chemically synthesized

<400>  115
agcatgaacg ccaaggaatg tacgttgaga atcactgctc caggcctgca ttactccttc        60

agctctgggg cagaggaagc                                                    80


<210>  116
<211>  80
<212>  DNA
<213>  Artificial sequence

<220>
<223>  chemically synthesized

<400>  116
aacatccagg ataaggaccg gatctctgcc atgcagagca tcttccagaa gaccaggact        60

ctgggaggcg aggagagctg                                                    80


<210>  117
<211>  80
<212>  DNA
<213>  Artificial sequence

<220>
<223>  chemically synthesized

<400>  117
ccttcccatc gcacaaccca aaaaagttat cacaccggaa agaagtcacc gagcgtctac        60

tccagaagtc agtatgtgta                                                    80

<210>  118
<211>  80
<212>  DNA
<213>  Artificial sequence

<220>
<223>  chemically synthesized

<400>  118
aactattaca tccttaacac cctctacccc aagttcaatg ataagttggc cgaaggcttc        60

ccccttcctc tgctgaagcg                                                    80


<210>  119
<211>  80
<212>  DNA
<213>  Artificial sequence

<220>
<223>  chemically synthesized

<400>  119
ggagactggt ttgaatatgt ttcttcccct aactacttag cagagctgat gatctacgtt        60

tccatggccg tcacctttgg                                                    80


<210>  120
<211>  80
<212>  DNA
<213>  Artificial sequence

<220>
<223>  chemically synthesized

<400>  120
attccgagtg gctgcctaca acaaagccaa aaaagccatg gacttctatg atccagcaag        60

gcacaatgag tttgacttca                                                    80


<210>  121
<211>  80
<212>  DNA
<213>  Artificial sequence

<220>
<223>  chemically synthesized

<400>  121
actattctct tgtttactgc cttttgactc ggatgaagag acacggaagg ggagaaatca        60

ttggaattca gaagctgaat                                                    80


<210>  122
<211>  80
<212>  DNA
<213>  Artificial sequence

<220>
<223>  chemically synthesized

<400>  122
aaagcttatg gctctgtgat gatattagtg accagcggag atgataagct tcttggcaat      60

tgcttaccca ctgtgctcag                                                  80


<210>  123
<211>  80
<212>  DNA
<213>  Artificial sequence

<220>
<223>  chemically synthesized

<400>  123
tggtcttgag agatggcttg ctggagaacc agaccccaga gttcttccag gacgtctgca      60

agcccaagta cagcggcacc                                                  80


<210>  124
<211>  80
<212>  DNA
<213>  Artificial sequence

<220>
<223>  chemically synthesized

<400>  124
gttgaagatg aaacagtaga gcttgatgtg tcagatgaag agatggctag aagatatgag      60

accttggtgg ggacaattgg                                                  80


<210>  125
<211>  80
<212>  DNA
<213>  Artificial sequence

<220>
<223>  chemically synthesized

<400>  125
ctgtgccaag cccttcaaag tcatctgtat cttcgtctct ttcctcagct ttgactacaa      60

actggtgcag aaggtgtgcc                                                  80


<210>  126
<211>  80
<212>  DNA
<213>  Artificial sequence

<220>
<223>  chemically synthesized

<400>  126
ctaatcttat gaacagtggt gtgagactga atgccatttg tccaggcttt gttaacacag      60

ccatccttga atcaattgaa                                                  80

<210>  127
<211>  80
<212>  DNA
<213>  Artificial sequence

<220>
<223>  chemically synthesized

<400>  127
ttggattaca ggagatgaga gtattgtagg ccttatgaag gacattgtag gagccaatga      60

gaaagaaata gccctaatga                                                  80


<210>  128
<211>  80
<212>  DNA
<213>  Artificial sequence

<220>
<223>  chemically synthesized

<400>  128
caggcactga acaatttggg gtttaagatt tgtccctgtg gctggcatca gtggaaatgc      60

acccccaaga aatgttgttg                                                  80


<210>  129
<211>  80
<212>  DNA
<213>  Artificial sequence

<220>
<223>  chemically synthesized

<400>  129
catgtcacca cgatctttcc tctgcttgag aagaccatgg aactggatag ctcacttccg      60

gaatacaaca tgtttccccg                                                  80


<210>  130
<211>  80
<212>  DNA
<213>  Artificial sequence

<220>
<223>  chemically synthesized

<400>  130
tgttcgagcc gcattctgga gaagactaca gtcgtgatga ggaccacatc gctcacatag      60

tggagcttct gggggacatc                                                  80


<210>  131
<211>  80
<212>  DNA
<213>  Artificial sequence

<220>
<223>  chemically synthesized

<400>  131
catcacatcc tcatgcacct caccgagaaa gcccaggagg tgacaaggaa ataccaggaa      60

atgacgggac aagtttggta                                                  80


<210>  132
<211>  80
<212>  DNA
<213>  Artificial sequence

<220>
<223>  chemically synthesized

<400>  132
tcgctgctgt gtcactcatg actgttgcta caaacgtctg gagaaacgtg gatgtggcac      60

caaatttctg agctacaagt                                                  80


<210>  133
<211>  80
<212>  DNA
<213>  Artificial sequence

<220>
<223>  chemically synthesized

<400>  133
cctgcactcc cgaaagaata tgctgttggc caccaagcaa ctttgtaaag aatttacgga      60

tctactggcg caggaccgga                                                  80


<210>  134
<211>  80
<212>  DNA
<213>  Artificial sequence

<220>
<223>  chemically synthesized

<400>  134
gcacattcaa gaacacggag attagtttcc agctgggaga agagtttgat gaaaccactg      60

cagatgatag aaactgtaag                                                  80


<210>  135
<211>  80
<212>  DNA
<213>  Artificial sequence

<220>
<223>  chemically synthesized

<400>  135
gtccttcaaa gctggagtct gtcctcctaa gaaatctgcc cagtgcctta gatacaagaa      60

acctgagtgc cagagtgact                                                  80

<210>  136
<211>  80
<212>  DNA
<213>  Artificial sequence

<220>
<223>  chemically synthesized

<400>  136
atgaaatgga gaacttgctg acctacaagc ggagagccat agagcacgtg ctgcaggtag        60

aggcctccca ggagccctcg                                                    80


<210>  137
<211>  80
<212>  DNA
<213>  Artificial sequence

<220>
<223>  chemically synthesized

<400>  137
atggaggacc tggacacaaa tgcagacaag cagctgagct tcgaggagtt catcatgctg        60

atggcgaggc taacctgggc                                                    80


<210>  138
<211>  80
<212>  DNA
<213>  Artificial sequence

<220>
<223>  chemically synthesized

<400>  138
agcttctcca gcagtgcggg tcctgggctc ctgaaggctt attccatccg gaccgcatcc        60

gccagtcgca ggagtgcccg                                                    80


<210>  139
<211>  80
<212>  DNA
<213>  Artificial sequence

<220>
<223>  chemically synthesized

<400>  139
agtggtgcac tcttctttat catctcagac ctgaccatcg ccctcaacaa attctgtttt        60

cctgtgccct actctcgggc                                                    80


<210>  140
<211>  80
<212>  DNA
<213>  Artificial sequence

<220>
<223>  chemically synthesized

<400>  140
ctgtctctta cacggtagca ccctttgtga tgttggcagt tgaaccgacc atcagcttat      60

acaagtccat gtacttttat                                                  80


<210>  141
<211>  80
<212>  DNA
<213>  Artificial sequence

<220>
<223>  chemically synthesized

<400>  141
tcctgtgctg ctgtctggtt gagagcctgc caccgtgtgt cgggagtgtg ggccaggctg      60

agtgcatagg tgacagggcc                                                  80


<210>  142
<211>  80
<212>  DNA
<213>  Artificial sequence

<220>
<223>  chemically synthesized

<400>  142
cctgctctgg atcatcgaac tgaataccaa cacaggcatc cgtaagaact tggagcagga      60

gatcatccag tacaacttta                                                  80


<210>  143
<211>  80
<212>  DNA
<213>  Artificial sequence

<220>
<223>  chemically synthesized

<400>  143
tgatggggag aatgtgaaaa ttccagtggc catcaaagtg ttgagggaaa acacatcccc      60

caaagccaac aaagaaatct                                                  80


<210>  144
<211>  80
<212>  DNA
<213>  Artificial sequence

<220>
<223>  chemically synthesized

<400>  144
attgaggcca tccgaagagc cagtaatgga gaaaccctag aaaagatcac caacagccgt      60

cctccctgcg tcatcctgtg                                                  80

<210>  145
<211>  80
<212>  DNA
<213>  Artificial sequence

<220>
<223>  chemically synthesized

<400>  145
ctcgatgcac acactggtat atcccatgaa gacctcatcc agaacttcct gaatgctggc        60

agctttcctg agatccaggg                                                    80


<210>  146
<211>  80
<212>  DNA
<213>  Artificial sequence

<220>
<223>  chemically synthesized

<400>  146
cctgacatgg actatgaccc tgaggcacga attctctgtg cgctgtatgt tgttgtctct        60

atcctgctgg agctggctga                                                    80


<210>  147
<211>  80
<212>  DNA
<213>  Artificial sequence

<220>
<223>  chemically synthesized

<400>  147
tcgtgctgta cttcctcacc agcttctaca ctaagtacga ccagatccat tttgtgctca        60

acaccgtgtc cctgatgagc                                                    80


<210>  148
<211>  80
<212>  DNA
<213>  Artificial sequence

<220>
<223>  chemically synthesized

<400>  148
acgatgtgca gccttcgaag ttgatgcgac tgctgagctc taatgaggac gatgccaaca        60

tcctttcgag ccccacagac                                                    80


<210>  149
<211>  80
<212>  DNA
<213>  Artificial sequence

<220>
<223>  chemically synthesized

<400>  149
tgtatcacat cacttctcaa gtattccttc attgggcttc atccttttag cagaactctt      60

ggtggtggga tagagactta                                                  80


<210>  150
<211>  80
<212>  DNA
<213>  Artificial sequence

<220>
<223>  chemically synthesized

<400>  150
atgaagatcg gaagaatcca gcatacatac ctcggaaagg gctcttcttt gagcatgatc      60

ttcgagggca aactcaggag                                                  80


<210>  151
<211>  80
<212>  DNA
<213>  Artificial sequence

<220>
<223>  chemically synthesized

<400>  151
tgtccggtct ttcttggatg attttgagtc aaagtattcc ttccatccag tagaagactt      60

tcctgctcca gaagaatata                                                  80


<210>  152
<211>  80
<212>  DNA
<213>  Artificial sequence

<220>
<223>  chemically synthesized

<400>  152
acatgactct aagtaacctc tgtgaccctc agttgaaacc agaagagaga gaatcttgta      60

accctcagga ctgtgtccct                                                  80


<210>  153
<211>  80
<212>  DNA
<213>  Artificial sequence

<220>
<223>  chemically synthesized

<400>  153
accttcctct cctaaacacg tctcttctgt cacttcccga actggcagtt ctggagcaaa      60

ggagatgaaa ctcaaggggg                                                  80

<210>  154
<211>  80
<212>  DNA
<213>  Artificial sequence

<220>
<223>  chemically synthesized

<400>  154
tgctggctcg gttatcagac tctcatccaa cacaagctca cagggaaagc cgttccttgc        60

tccttgtgga gggagctacc                                                    80


<210>  155
<211>  80
<212>  DNA
<213>  Artificial sequence

<220>
<223>  chemically synthesized

<400>  155
gcatctgagc agtgaggaga tgatgagaga atgtggagat gtgtcaaggg ctcaagagga        60

gctaaggaaa aaacagaaaa                                                    80


<210>  156
<211>  80
<212>  DNA
<213>  Artificial sequence

<220>
<223>  chemically synthesized

<400>  156
aattcttcga ggacatttta cagccagaga cagagtttgt ctttcctctg tcccatctgc        60

atctcgagtc gcagagaccc                                                    80


<210>  157
<211>  80
<212>  DNA
<213>  Artificial sequence

<220>
<223>  chemically synthesized

<400>  157
ccaggtgcag gatatcgtca agccaaacac agcacataca ttcatcataa caggaagaaa        60

aaggtccctg gagctgcaga                                                    80


<210>  158
<211>  80
<212>  DNA
<213>  Artificial sequence

<220>
<223>  chemically synthesized

<400>  158
gtctcggtgc ttccagccac ataaccacaa aatcaatggc tgctcctcca agctgtggtc      60

tgagcccact gctggtcctg                                                  80


<210>  159
<211>  80
<212>  DNA
<213>  Artificial sequence

<220>
<223>  chemically synthesized

<400>  159
ctgggcacta tggaggtgaa gagcaagttt ggagctgaat ttcgtcggtt ttcgctggaa      60

agatcaaaac ctggaaaatt                                                  80


<210>  160
<211>  80
<212>  DNA
<213>  Artificial sequence

<220>
<223>  chemically synthesized

<400>  160
gtgcaggttc agtgtgagca gaactgggga agcctgtgct ccatcttgag cttctgcacc      60

tcggccatcc agaagcctcc                                                  80


<210>  161
<211>  80
<212>  DNA
<213>  Artificial sequence

<220>
<223>  chemically synthesized

<400>  161
ctgttgctgt gttctgtcat gagttgcctc atgaattagg tgactttgct gttctactaa      60

aggctggcat gaccgttaag                                                  80


<210>  162
<211>  80
<212>  DNA
<213>  Artificial sequence

<220>
<223>  chemically synthesized

<400>  162
caatatcacc gccatgccac acaatggatc attctggaat gtccaggatc tgctcaattc      60

agcaatgcca agggtggtcc                                                  80

<210>  163
<211>  80
<212>  DNA
<213>  Artificial sequence

<220>
<223>  chemically synthesized

<400>  163
tcgctgtcct tgcggaatcc acacactttg ggcctaagcc ggtaccaccc ctatggcaag        60

agccacttat ccacagcggg                                                    80


<210>  164
<211>  80
<212>  DNA
<213>  Artificial sequence

<220>
<223>  chemically synthesized

<400>  164
gcaagtaaag aagaggcaac cacgttctcc aatgcaatgc tgtttgccct gaacatcatg        60

aattcccaag aaggaggccc                                                    80


<210>  165
<211>  80
<212>  DNA
<213>  Artificial sequence

<220>
<223>  chemically synthesized

<400>  165
ggtgcagatg atggctttgt tacggtgagc ctaaagccag acaaagggaa gagagcaaat        60

tcccaagaaa atcgtaatta                                                    80


<210>  166
<211>  80
<212>  DNA
<213>  Artificial sequence

<220>
<223>  chemically synthesized

<400>  166
cgctgaaaca cgtccatttg gagaacaacc gcttgaacca gctaccctcg aacttcccct        60

tcgacagcct ggagaccctc                                                    80


<210>  167
<211>  80
<212>  DNA
<213>  Artificial sequence

<220>
<223>  chemically synthesized

<400>  167
tggcctttca ttgtgatctc tgatgactcc tgcgtgatgt ggaacgtggt ggatgtcaac      60

tctgctgggg agagaagcag                                                  80


<210>  168
<211>  80
<212>  DNA
<213>  Artificial sequence

<220>
<223>  chemically synthesized

<400>  168
ataatcagca atgatgtctg caatggcgct gacttctatg gaaaccagat caagcccaag      60

atgttctgtg ctggctaccc                                                  80


<210>  169
<211>  80
<212>  DNA
<213>  Artificial sequence

<220>
<223>  chemically synthesized

<400>  169
cggtgatggt attttgccca ggcaacagta cttcaaacag aactgcagtc agcatgaatc      60

cagtccagat atttcacatt                                                  80


<210>  170
<211>  80
<212>  DNA
<213>  Artificial sequence

<220>
<223>  chemically synthesized

<400>  170
ccagcttctg cagtttctgt tttgaagagc cagcacttac ggcagaaact tcttcagtct      60

ctgtttcttg ataaagtgta                                                  80


<210>  171
<211>  80
<212>  DNA
<213>  Artificial sequence

<220>
<223>  chemically synthesized

<400>  171
cacaggattc ctgtgggtcc agctttggaa ctgggaaacc tttcttcgga tccgcactca      60

ttccactgat gccagctgcc                                                  80

<210>  172
<211>  80
<212>  DNA
<213>  Artificial sequence

<220>
<223>  chemically synthesized

<400>  172
tctacattct ggttgtcatg tccttctacg gcattttctt gatcggaatc atgctgggct      60

acatgaaatc caagaggcgg                                                  80


<210>  173
<211>  80
<212>  DNA
<213>  Artificial sequence

<220>
<223>  chemically synthesized

<400>  173
tggtggagat cttcgacatg ctgctggcta catcatctcg gttccgcatg atgaatctgc      60

agggagagga gtttgtgtgc                                                  80


<210>  174
<211>  80
<212>  DNA
<213>  Artificial sequence

<220>
<223>  chemically synthesized

<400>  174
atgaagaatt tcttcattct gatctcctag aagacagcaa ataccgaaaa atctactcct      60

ttactcttaa gcctcgaaca                                                  80


<210>  175
<211>  80
<212>  DNA
<213>  Artificial sequence

<220>
<223>  chemically synthesized

<400>  175
ctgagcactg ctctccgtga cccacactac tttgaaaaac cagacgcctt caatcctgac      60

cactttctgg atgccaatgg                                                  80


<210>  176
<211>  80
<212>  DNA
<213>  Artificial sequence

<220>
<223>  chemically synthesized

<400>  176
aaaatgggag ttttctgttc catttcatca ggctctgaat ggcttccatc ctgcaccact      60

ctgtaccata agaacattaa                                                  80


<210>  177
<211>  80
<212>  DNA
<213>  Artificial sequence

<220>
<223>  chemically synthesized

<400>  177
tttgggctct tcgctggcct tggtggagct ggtgccagca ccagtggcag ctctggtgcc      60

tgtggtttct cctacaagtg                                                  80


<210>  178
<211>  80
<212>  DNA
<213>  Artificial sequence

<220>
<223>  chemically synthesized

<400>  178
accagacgga aaacccgttt ctgtgcacag tggttgccat cctcctccac tacatctaca      60

tgagcacctt tgcctggacc                                                  80


<210>  179
<211>  80
<212>  DNA
<213>  Artificial sequence

<220>
<223>  chemically synthesized

<400>  179
tggagacttc atcctgggct actatagtca caaccacagc atcctcatcg gcatcactga      60

acccttccag atctcgctgc                                                  80


<210>  180
<211>  80
<212>  DNA
<213>  Artificial sequence

<220>
<223>  chemically synthesized

<400>  180
tgagcagcaa gcgaatcacc atcaacaaga ttctgtccaa cgagagcctt gtgcaggaga      60

acctgtactt ccagcgctgc                                                  80

<210>  181
<211>  80
<212>  DNA
<213>  Artificial sequence

<220>
<223>  chemically synthesized

<400>  181
ccctctggat gagtccgaga gagatggagg ctctgaggac caagtggaag acccagcact        60

aagtgagcct ggggaggaac                                                    80


<210>  182
<211>  80
<212>  DNA
<213>  Artificial sequence

<220>
<223>  chemically synthesized

<400>  182
aaggcaatgg ctgtgcccta tcttctgaga agaaagttca gtaattccct gaaaagtcaa        60

ggtaaagatg atgattcttt                                                    80


<210>  183
<211>  80
<212>  DNA
<213>  Artificial sequence

<220>
<223>  chemically synthesized

<400>  183
ctcggagcag cagcataagc tggacttcaa ggcatacgaa caggcactgc aatactcgcc        60

ttacggctct acgttgcccg                                                    80


<210>  184
<211>  80
<212>  DNA
<213>  Artificial sequence

<220>
<223>  chemically synthesized

<400>  184
cagattcaga gtctgatatc ctgttgggca ttctggacaa cttggaccca gtcatgttct        60

tcaaatgccc ttccccagag                                                    80


<210>  185
<211>  80
<212>  DNA
<213>  Artificial sequence

<220>
<223>  chemically synthesized

<400>  185
aatgtctagc aaatccaaaa agtgcaaaaa agtgcatgac tcactggagg acttccccaa      60

gaacagctcg tttaacccgg                                                  80


<210>  186
<211>  80
<212>  DNA
<213>  Artificial sequence

<220>
<223>  chemically synthesized

<400>  186
gggcatctat ggccagtatc aactccttgg catcaagagc attggctttg aatggaatta      60

tccactagag gagccgacca                                                  80


<210>  187
<211>  80
<212>  DNA
<213>  Artificial sequence

<220>
<223>  chemically synthesized

<400>  187
tgggcaagta tattctgctc atcaaatggt cattggctat gtactttgtg cagggaagta      60

cattatctac agtcacaaaa                                                  80


<210>  188
<211>  80
<212>  DNA
<213>  Artificial sequence

<220>
<223>  chemically synthesized

<400>  188
cctttactgc ccctgcttaa cacggtcaag ttcctgctgc ttttctacct gaagaagctt      60

accctcttct ccacctgctc                                                  80


<210>  189
<211>  80
<212>  DNA
<213>  Artificial sequence

<220>
<223>  chemically synthesized

<400>  189
tatgcttacg aacctgcaga tacagctctg ttgcttgaca acatgaagaa agctctcaag      60

ttgctgaaga ctgaattgta                                                  80

<210>  190
<211>  80
<212>  DNA
<213>  Artificial sequence

<220>
<223>  chemically synthesized

<400>  190
ccaggatccc tggagtgcct tggtgtttca agcccctgac taggaagaca gaatgcacct        60

tctgaggcac ctccagctgc                                                    80


<210>  191
<211>  80
<212>  DNA
<213>  Artificial sequence

<220>
<223>  chemically synthesized

<400>  191
tgccatagat gaattgaagg aatgttttct taaccaaacg gatgaaactc tgagcaatgt        60

tgaggtgttt atgcaattaa                                                    80


<210>  192
<211>  80
<212>  DNA
<213>  Artificial sequence

<220>
<223>  chemically synthesized

<400>  192
gcttctacca ctgctcgtaa agacattcca gttttaccca aatgggttgg ggatctcccg        60

aatggtagag tgtgtccctg                                                    80


<210>  193
<211>  80
<212>  DNA
<213>  Artificial sequence

<220>
<223>  chemically synthesized

<400>  193
acctgttcaa ggtgcagcga tactgtgaga agagcatgat cagcagaaag ctctttggat        60

tcactgaacg gtacggagca                                                    80


<210>  194
<211>  80
<212>  DNA
<213>  Artificial sequence

<220>
<223>  chemically synthesized

<400>  194
cagctcatca ctgttgaaaa ggctttggca attctttctc agcctacacc ctcacttgtt      60

gtggatcatg agcgattaaa                                                  80


<210>  195
<211>  80
<212>  DNA
<213>  Artificial sequence

<220>
<223>  chemically synthesized

<400>  195
catccagaga aagcacctta tgagtggaag aatttggatg gcatttccca tgcacctaat      60

gctgtgaaaa ccgcatttta                                                  80


<210>  196
<211>  80
<212>  DNA
<213>  Artificial sequence

<220>
<223>  chemically synthesized

<400>  196
accctgagac tcccagatct tcaggttcta tgcgcaatag atggaaacca aacagcagca      60

aggtactagg gagatccccc                                                  80


<210>  197
<211>  80
<212>  DNA
<213>  Artificial sequence

<220>
<223>  chemically synthesized

<400>  197
agcactctac acagtcacgg gacaaagctg gcctgaatca ttaatacgaa agactggata      60

taccctggaa agtcttaagc                                                  80


<210>  198
<211>  80
<212>  DNA
<213>  Artificial sequence

<220>
<223>  chemically synthesized

<400>  198
gaataagtat gcaagcagca aactcctgaa gatcagcatg atccctcagc tgaactcaaa      60

agccgtcaaa gaccttgcct                                                  80

<210>  199
<211>  80
<212>  DNA
<213>  Artificial sequence

<220>
<223>  chemically synthesized

<400>  199
ctctgtgtta ccaagaactg gctgtctgca gatactaaag aagagcggga tctctggatg        60

caaaaactca atcaagttct                                                    80


<210>  200
<211>  80
<212>  DNA
<213>  Artificial sequence

<220>
<223>  chemically synthesized

<400>  200
gctgccgagc tctggaaaaa ccccacagct tttaagaagt acctgcaaga aacctactca        60

aagcaggtca ccagccagga                                                    80


<210>  201
<211>  80
<212>  DNA
<213>  Artificial sequence

<220>
<223>  chemically synthesized

<400>  201
tgggttcatt acatgattca tgagccagaa ccacatattc ttctctttag acgacctctt        60

ccaaaagatc aacaaaaatg                                                    80


<210>  202
<211>  80
<212>  DNA
<213>  Artificial sequence

<220>
<223>  chemically synthesized

<400>  202
agcctgcagc aagcactttg gagagcaaat ctgtgcagag agtaacgcgg agtgtcaaga        60

ggtgtgcaga aaatccaaag                                                    80


<210>  203
<211>  80
<212>  DNA
<213>  Artificial sequence

<220>
<223>  chemically synthesized

<400>  203
gaaggcctgg tcctggacac aatgaatgac agcctcagca agatcctgct ggacatcagc      60

tttcctggcc tggacgagga                                                  80


<210>  204
<211>  80
<212>  DNA
<213>  Artificial sequence

<220>
<223>  chemically synthesized

<400>  204
gacagagaag catgcaagac agaaacaaaa ggctgatgag gaagagatgc ttgataatct      60

accagaggct ggtgactcca                                                  80


<210>  205
<211>  80
<212>  DNA
<213>  Artificial sequence

<220>
<223>  chemically synthesized

<400>  205
gaatggtttc tgcataccct cgacagctag agtcattaat ccgcttagca gaagcccatg      60

ctaaagtaag attgtctaac                                                  80


<210>  206
<211>  80
<212>  DNA
<213>  Artificial sequence

<220>
<223>  chemically synthesized

<400>  206
taagagatca ttatccttgt gtgtcttcat tggaacagct aacggagttg ggaagagaaa      60

ctgatgcttt tgtatgccgc                                                  80


<210>  207
<211>  80
<212>  DNA
<213>  Artificial sequence

<220>
<223>  chemically synthesized

<400>  207
ggactgcaga gaaggaactc ctctgaagca agctccggag acttcttaga tctgaaggga      60

gaaggtgata ttcatgaaaa                                                  80

<210>  208
<211>  80
<212>  DNA
<213>  Artificial sequence

<220>
<223>  chemically synthesized

<400>  208
ctgtagagga agaggaagat caaaacgttg taccagaaac tacctctgaa ggctacactt      60

tccaagttca ggatggggct                                                  80


<210>  209
<211>  80
<212>  DNA
<213>  Artificial sequence

<220>
<223>  chemically synthesized

<400>  209
ggcttctgaa ctggaattct gcagctaacc cttccacgac tagaacctta ggcattgggg      60

agttttagat ggactaattt                                                  80


<210>  210
<211>  80
<212>  DNA
<213>  Artificial sequence

<220>
<223>  chemically synthesized

<400>  210
atctcaggac tctaaatact ctaacagctg tccagtgttg gtgattccag tggactgtat      60

ctctgtgaaa aacacaattt                                                  80


<210>  211
<211>  80
<212>  DNA
<213>  Artificial sequence

<220>
<223>  chemically synthesized

<400>  211
catttggagt ataggcacca tatttgctga actagcaact aagaaaccac ttttccatgg      60

ggattcagaa attgatcaac                                                  80


<210>  212
<211>  80
<212>  DNA
<213>  Artificial sequence

<220>
<223>  chemically synthesized

<400>  212
tccaatctgt tgcagtctcc ttcaagcatt ctgtcgaccc tggatgttga attgccacct      60

gtttgctgtg acatagatat                                                  80


<210>  213
<211>  80
<212>  DNA
<213>  Artificial sequence

<220>
<223>  chemically synthesized

<400>  213
cgcccaccaa gaaggaacat cagaaagcct gggctttgaa cctgaacggt tttgatgtag      60

aggaagccaa gatccttcgg                                                  80


<210>  214
<211>  80
<212>  DNA
<213>  Artificial sequence

<220>
<223>  chemically synthesized

<400>  214
gaggaagacc aaaaagtcat cgagctggtt aagaagtatg gcacaaagca gtggacactg      60

attgccaagc acctgaaggg                                                  80


<210>  215
<211>  80
<212>  DNA
<213>  Artificial sequence

<220>
<223>  chemically synthesized

<400>  215
tttgtggcag acagacttat gctggaactg ggttttagca aggttttcag agtagagaac      60

ccatttgact ttatggagaa                                                  80